(19) Europäisches Patentamt
European Patent Office
Office européen des brevets

(11) Publication number: **0 172 919 A1**

# (12) EUROPEAN PATENT APPLICATION

published in accordance with Art. 158(3) EPC

(21) Application number: **85901085.2**

(22) Date of filing: **01.03.85**

Data of the international application taken as a basis:

(86) International application number: **PCT/JP 85/00101**

(87) International publication number: **WO 85/03938 (12.09.85 85/20)**

(51) Int. Cl.[4]: **C 07 D 519/00**, C 07 D 501/46, A 61 K 31/545

(30) Priority: **05.03.84 JP 40503/84**
**19.07.84 JP 148544/84**

(43) Date of publication of application: **05.03.86**
**Bulletin 86/10**

(84) Designated Contracting States: **AT BE CH DE FR GB LI LU NL SE**

(71) Applicant: **TEIJIN LIMITED, 11 Minamihonmachi 1-chome Higashi-ku, Osaka-shi Osaka 541 (JP)**

(72) Inventor: **ICHIKAWA, Yataro 11-7, Kotesashi-machi 2-chome, Tokorozawa-shi, Saitama 359 (JP)**
Inventor: **YOSHISATO, Eishin 110-22, Minamiiwakuni-machi, 2-chome Iwakuni-shi, Yamaguchi 740 (JP)**
Inventor: **HARADA, Toshiaki 69-5, Ushinoya-machi 3-chome, Iwakuni-shi, Yamaguchi 741 (JP)**
Inventor: **IMAI, Hiroshi 9-2, Yamate-cho 2-chome, Iwakuni-shi, Yamaguchi 740 (JP)**
Inventor: **SUZUKI, Yoji 20-2, Tamadaira 5-chome, Hino-shi, Tokyo 191 (JP)**
Inventor: **MIYANO, Seiji 6-13, Nagaoka 2-chome, Minami-ku, Fukuoka-shi, Fukuoka 815 (JP)**
Inventor: **SUMOTO, Kunihiro 4-65, Tsutsujigaoka 2-chome, Oonojyo-shi, Fukuoka 816 (JP)**

(74) Representative: **Votier, Sidney David et al, CARPMAELS & RANSFORD 43, Bloomsbury Square, London WC1A 2RA (GB)**

## (54) CEPHALOSPORIN DERIVATIVES, PROCESS FOR THEIR PREPARATION, AND THEIR PHARMACEUTICAL USE.

(57) Cephalosporin derivatives represented by formula (I), (wherein $R^1$ represents a hydrogen atom or a protective group, $R^2$ represents a hydrogen atom, a substituted or unsubstituted alkyl group, a substituted or unsubstituted cycloalkyl group, or a substituted or unsubstituted heterocyclic group, $R^3$ represents a hydrogen atom, a substituted or unsubstituted alkyl group, a cyano group, a carboxy group, an alkoxycarbonyl group, a carbomoyl group, a carbanolyloxy group, a heterocyclic ring, a hydroxy group, an alkoxy group or a halogen atom, $R^4$ and $R^5$ may be the same or different and each represents hydrogen or alkyl, l represents 0, 1 or 2, m and n each represents 1 or 2 when l is O, or represents O when l is 1 or 2, and X represents methylene when l is O or 1 or represents methylene, substituted methylene or oxo when l is 2, provided that X does not represent methylene when $R^3$, $R^4$, and $R^5$ all represent hydrogen), salts or esters thereof have strong antibacterial activity, and are useful for treatment and prevention of various bacteria-induced diseases.

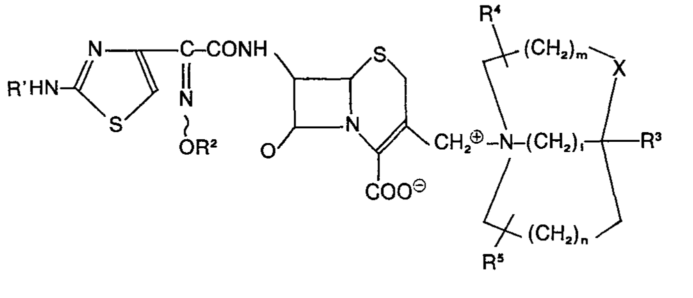

(1)

EP 0 172 919 A1

SPECIFICATION

TITLE OF THE INVENTION

CEPHALOSPORIN DERIVATIVE, PROCESS FOR PRODUCTION THEREOF AND PHARMACEUTICAL USE THEREOF

## TECHNICAL FIELD

The present invention relates to a cephalosporin derivative, a process for the production thereof, and the pharmaceutical use thereof. More particularly, this invention relates to a novel cephalosporin derivative which exhibits a high antibacterial activity and strongly inhibits the growth of a number of microorganism including Gram-positive and Gram-negative bacteria, a process for the production thereof, and the pharmaceutical use thereof.

## BACKGROUND OF THE ART

The cephalosporin derivatives which have a 2-methoxyimino-2-(2-aminothiazol-4-yℓ)-acetoamido group at the 7-position of their cephalosporin skeleton are widely known as the third-generation antibiotics and looked upon as the important remedy for various types of infectious deseases in the present medical field.

As the like cephalosporin derivatives, there are, for instance, a cephalosporin derivative, known under the name of Cefotaxime, which has an acetoxymethyl group at the 3-position, and is characterized by its stability against β-lactamase, and another known cephalosporin derivative (Cefmenoxime) which has a strong antibiotic activity against

Gram-negative and Gram-positive bacteria.

Apart from those mentioned above, cephalosporin derivatives which have a quaternary ammonium methyl group at the 3-position are known. For instance, U.S. Patent No. 4,278,671 discloses cephalosporin derivatives which have such a quaternary ammonium methyl group as pyridiniummethyl, quinoliniummethyl, and picoliniummethyl. Also, Japanese Laid-Open Patent Pubulication No. 219292/1984 contains cephalosporin derivatives which have such a quaternary ammonium methyl group as 1-(1-azabicyclo-[2,2,2]-octane-1-io)methyl group at the 3-position.

However, cephalosporin derivatives which have such a specific quaternary ammonium methyl group such as 1-(1-azabicyclo-[3,3,0]octane-1-io)methyl group have hitherto not been known.

## DISCLOSURE OF THE INVENTION

One of the objects of this invention is to provide a novel cephalosporin derivative which has a specific quarternary ammonium methyl group such as 1-(1-azabicyclo-[3,3,0]-octane-1-io)methyl group at the 3-position.

Another object of this invention is to provide a novel cephalosporin derivative which has a strong antibiotic activity.

A further object of this invention is to provide a novel cephalosporin derivative which has a strong antibiotic activity against a number of Gram-positive and Gram-negative bacterias.

Still another object of this invention is to provide a novel cephalosporin derivative which has a strong antibiotic activity especially against Gram-negative such as Pseudomonas aeruginosa, Salmonera

thyphosa and or Gram-positive such as Staphyrococcus, Streptococcus and Butilus subtilus.

A further object of this invention is to provide a novel cephalosporin derivative which has a long duration of antibiotic activity.

Yet another ojbect of this invention is to provide a process for the production of a novel cephalosporin derivative and its pharmaceutical use.

Other objects and advantages of this invention will become apparent from the following description.

In accordance with this invention, these objects and advantages are achieved by a cephalosporin derivative of the formula (1)

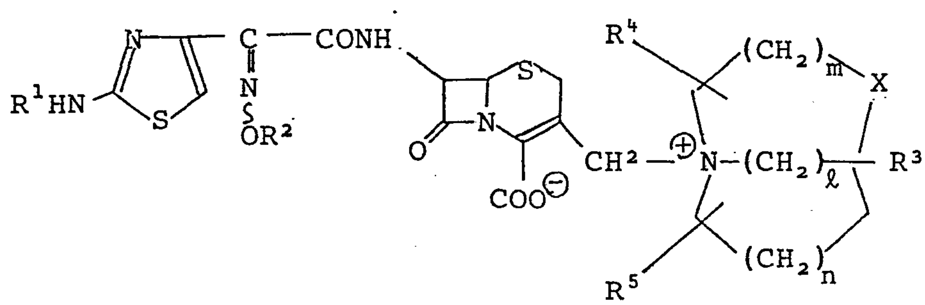

(1)

wherein $R^1$ represents a hydrogen atom or a protective group; $R^2$ represents a hydrogen atom, substituted or unsubstituted alkyl group, a substituted or unsubstituted cycloalkyl group, or a substituted or a unsubstituted hereto ring group; $R^3$ represents a hydrogen atom, a substituted or unsubstitued alkyl group, a cyano group, a carboxyl group, an alkoxycabonyl group, a carbamoyl group, a carbamoyloxy group, a hetero ring group, a hydroxyl group, an alkoxyl group or a halogen atom;

$R^4$ and $R^5$ are idential or different and each represents a hydrogen atom or an alkyl group; ℓ represents 0, 1 or 2; m and n respectively represent 1 or 2 when ℓ is 0; m and n respectively represent 0 when ℓ is 1 or 2; X represents a methylene group when ℓ is 0 or 1; and X represents a methylene group, a methylene group having a substituent, or an oxo group when ℓ is 2, provided that when $R^3$, $R^4$ and $R^5$ respectively represent a hydrogen atom, X is not a methylene group

or its salt or ester thereof.

BEST MODE OF CARRYING OUT THE INVENTION

The cephalosporin derivative expressed by the formula (1) can be classified into the undermentioned three types of molecules according to the definitions of ℓ, m, n and X.

(1-1) <u>Cephalosporin derivative in which ℓ is 0, m and n are 1 or 2 and X is a methylene group:</u>

In this case, the cepharosporin derivative of this invention is expressed by the formula (1-1)

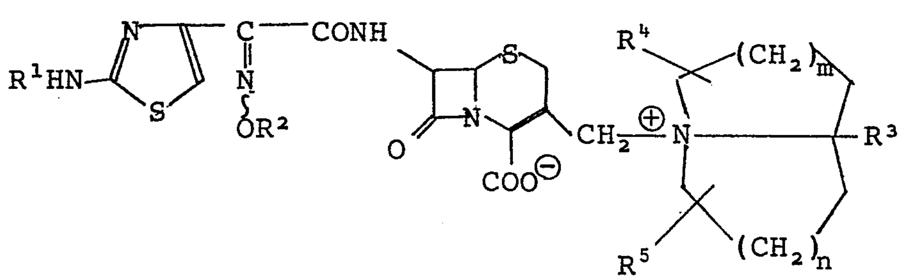

(1-1)

wherein $R^1$, $R^2$, $R^3$, $R^4$, $R^5$, m and n are as defined above.

In the formula (1-1), m and n respectively represent 1 or 2.

(1-2) Cephalosporin derivative in which $\ell$ is 1, m and n are 0 and X is a methylen group

In this case, the cephalosporin derivative of this invention is expressed by the formula (1-2)

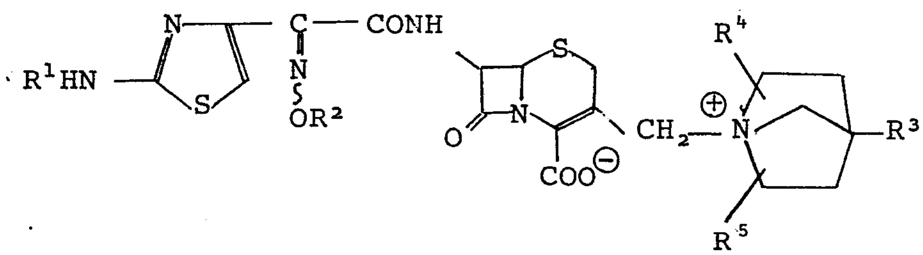

(1-2)

wherein $R^1$, $R^2$, $R^3$, $R^4$ and $R^5$ are as defined above.

(1-3) Cephalosporin derivative in which $\ell$ is 2, m and n are 0, and X is a methylene group, a methylene group having a substituent, or an oxo group, provided that when $R^3$, $R^4$, and $R^5$ respectively represent a hydrogen group, X is not a methylene group.

In this case, the cephalosporin derivative of this invention is expressed by the formula (1-3)

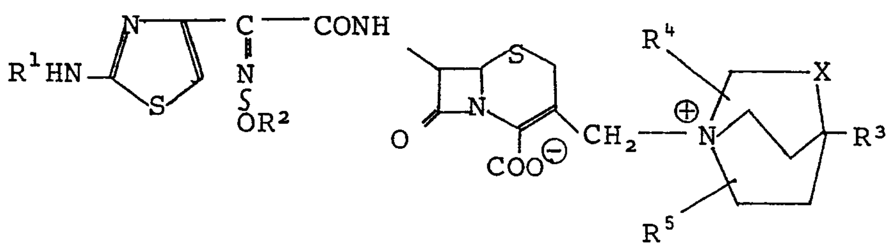

(1-3)

wherein $R^1$, $R^2$, $R^3$, $R^4$ and $R^5$ are

as defined above and X represents a methylene group, a methylene group having a substituent, or an oxo group, provided that when $R^3$, $R^4$ and $R^5$ respectively represents a hydrogen atom, X is not a methylene group.

In the formula (1-3), X is a methylene group, a methylene group having a substituent, or an oxo group, provided that when $R^3$, $R^4$ and $R^5$ respectively represents a hydrogen atom, X is not a methylene group.

Examples of the substituent of the said methylene group include a hydroxyl group; an amino group; a hologen atom such as chlorine, bromine and iodine; and an alkyl group such as methyl, ethyl, propyl, isopropyl, butyl, tert-butyl, pentyl and hexyl.

Referring to the aforementioned formula (1), (1-1), (1-2), and (1-3), said cephalosporin derivatives of formula (1), (1-1), (1-2), and (1-3) have a partial structure of 2-aminothiazole-4-yℓ expressed by the following formula respectively:

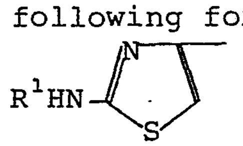

Said partial structure can be easily isomerized to 2-aminothiazoline-4-yℓ expressed by the formula:

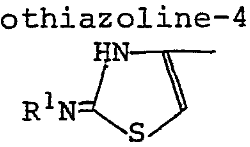

Therefore, the cephalosporin derivatives of formula (1), (1-1), (1-2), and (1-3) may contain any of thier tautomers and this invention involves all such tautomers.

The cephalosporin derivatives of the respective formula (1), (1-1), (1-2), and (1-3) have a partial structure expressed by the following formula

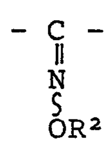

and this partial structure may be made up of a syn form expressed by formula

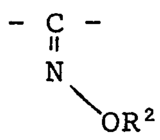

or of anti form expressed by the formula

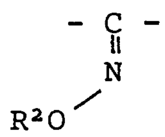

or of mixture of these isomers combined in any proportion.

Among the cephalosporin derivatives of this invention, those of a syn form are preferable because of their strong antibiotic activity.

In the formula (1), (1-1), (1-2) and (1-3), $R^1$ represents a hydrogen atom or a protective group.

The protective group of $R^1$ may include an acyl group and an aralkyl group which may be substituted.

Suitable examples of an acyl group are lower alkanoyl such as formyl, acetyl, propionyl, butyryl, isobutyryl, valeryl, isovaleryl, oxalyl, succinyl and pivaloyl; lower alkoxycarbonyl such as methoxycarbonyl, ethoxycarbonyl, propoxycarbonyl, isopropoxycarbonyl, butoxycarbonyl and pentyloxycarbonyl; lower alkanesulfonyl such as mesyl, ethanesulfonyl, propanesulfonyl, isopropanesulfonyl and butanesulfonyl; aroyl such as benzoyl, toluoyl, naphthoyl and phthaloyl; aralkanoyl such as phenylacetyl and phenylpropionyl; and araalkoxycarbonyl such as benzyloxycarbonyl.

The acyl group as stated above may have at least one suitable substituent. The substituent may, for example, include halogen atom such as chlorine, bromine, iodine and fluorine; cyano; and lower alkyl such as methyl, ethyl, propyl and butyl.

Suitable examples of an aralkyl group which may be substituted are benzyl, 4-methoxybenzyl, phenethyl, trityl and 3,4-dimethoxybenzyl.

In the formula (1), (1-1), (1-2) and (1-3), $R^2$ represents a hydrogen atom, substituted or unsubstituted alkyl group, a substituted or unsubstituted cycloalkyl group, or s substituted or unsubstituted hetero ring group. Examples of the unsubstituted alkyl group are linear or branched $C_1$- $C_{10}$ alkyl groups such as methyl, ethyl, propyl, iso-propyl, butyl, sec-butyl, tertbutyl, pentyl, hexyl, n-heptyl, octyl, nonyl and decyl.

The substituent of the said alkyl group, for examples, include carboxyl group; alkoxycarbonyl group such as menthoxycarbonyl, ethoxycarbonyl, tert-butoxycarbonyl and pentoxycarbonyl; hetero ring atom such as imidazol, 5-methylimidazol, 1-methylimidazol, 2-methyl-5-nitro-3-imidazol, 2-aminothiazol, 1H-tetrazol, 2H-tetrazol, thiazol, 1-methyl-1H-tetrazol and pyrolidone-3-yl.

Examples of the unsubstituted cycloalkyl group are $C_3$-$C_6$ cycloalkyl groups such as cyclopropyl, cyclobutyl, cyclopentyl and cyclohexyl.

Examples of the substituent of the unsubstitutued cycloalkyl group may include the same substituents as exemplified in substituent of the unsubstituent alkyl group.

Examples of the unsubstituted hetero ring group are 1H-tetrazol, 2H-tetrazol, pyrolidone-3-yl and 2-aminothiazol. Examples of the substituent of the unsubstituted hetero ring group may include alkyl group such as methyl, ethyl, propyl and butyl; alkoxyl group such as methoxy, ethoxy, propoxy and butoxy; and halogen atom such as florine, bromine and chorine.

$R^2$ is preferably a lower alkyl group such as methyl, ethyl, propyl, iso-propyl, butyl, tertbutyl

and hexyl, and a lower alkyl group substituted by a carboxyl group such as carboxyl methyl, 2-carboxyletyyl, 2-carboxy-2-propyl, 2-carboxy-2-butyl and 3-carboxy-3-pentyl.

In the formula (1), (1-1), (1-2) and (1-3), $R^3$ represent a hydrogen atom, a substituted or unsubstituted alkyl group, a cyano group, a carboxyl group, an alkoxycarbonyl group, a carbamoyl group, a carbamoyloxy group, a hetero ring roup, a hydroxyl group, an alkoxyl group or a halogen atom.

Examples of the unsubstituted alkyl group are linear or branched $C_1$- $C_6$ alkyl groups such as methyl, ethyl, propyl, isopropyl, butyl, sec-butyl, tert-butyl, pentyl and hexyl. The substituent of the said alkyl group, for example, includes hydroxyl group, cyano group; carbamoyloxy group; $C_1$-$C_6$ lower alkoxy group such as methoxy, ethoxy, butoxy, pentoxy and hexoxy carboxyl group; and hetero ring group such as 2,5-dihydro-2-methyl-5-oxoteriazin-3-yl and 2,5-dihydro-6-hydroxy-2-methyl-5-oxotriazin-3-yl.

Examples of the alkoxycarbonyl group are methoxycarbonyl, ethoxycarbonyl, propoxycarbonyl, butoxycarbonyl, pentoxycarbonyl, or hexoxycarbonyl.

The hetero ring group, for example, include 2,5-hihydro-2-methyl-5-oxotriazin-3-yl, 2,5,-dihydro-6-hydroxy-2-methyl-5-oxotriazin-3-yl, and 2-aminothiazol-4-yl.

Examples of the alkoxyl group are methoxy, ethoxy, propoxy, butoxy and pentoxy. Examples of the halogen atom are chlorine, bromine or iodine.

Of these, $R^3$ is preferably a hydrogen atom, a substituted or unsubstituted methyl group, a cyano group or an alkoxycarbonyl group.

In the formula (1), (1-1), (1-2) and (1-3), $R^4$ and $R^5$ are indentical or different and each represents a hydrogen atom or an alkyl group.

Examples of the alkyl group are linear or branched $C_1$-$C_6$ alkyl groups such as methyl, ethyl, propyl, iso-propyl, butyl, sec-butyl, tert-butyl, pentyl and hexyl.

$R^4$ and $R^5$ are preferably a hydrogen atom respectively.

Though cephalosporin derivatives of the formula (1), (2-1), (1-2) and (1-3) take a betaine structure, the cephalospirin derivatives of this invention may be salt drived from these betaine structures.

As such salts, acid addition salts may be mentioned. Examples of the salt may include inorganic acid salt such as hydrochloride, hydrobromide, sulfate and phosphate; organic acid salt such as acetate, trichloroacetate, trifluoroacetate, maleate, tartrate, methanesulfonate, benzensulfonate and toluenesulfonate; and a salt with an amino acid such as orginine, aspartic acid and glutamic acid.

These acid addition salts are presented by the following formulae (1') and (1'').

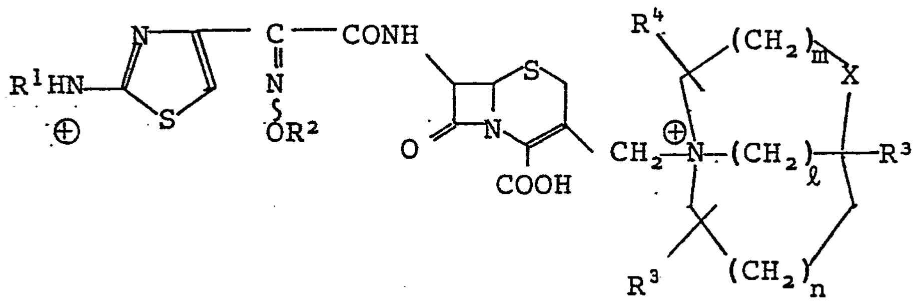

(1')

$An_1^{\ominus}$, $\frac{1}{2}An_2^{2\ominus}$ or $\frac{1}{3}An_3^{3\ominus}$

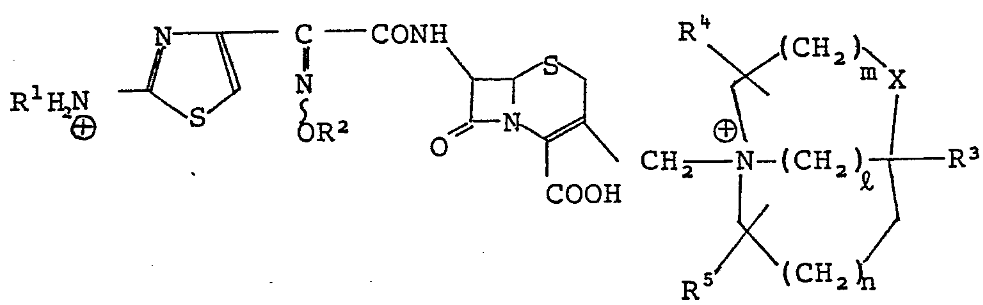

(1'')

$2An_1^{\ominus}$, $An_2^{2\ominus}$ or $\frac{2}{3}An_3^{3\ominus}$

wherein $R^1$, $R^2$, $R^3$, $R^4$, $R^5$, $\ell$, m, n and X are as defined above, $An_1^-$ is an anion of monobasic acid, $An_2^{2-}$ is an anion of dibasic acid, and $An_3^{3-}$ is an anion of tribasic acid.

In formulae (1') and (1''), An represents an anion of monobasic acid, such as $C\ell^-$, $Br^-$, $CH_3COO^-$, $CC\ell COO^-$ and $CF_3COO^-$, $An^{2-}$ represents an anion of dibasic acid such as $SO_4^{2-}$ and $An_3^{3-}$ represents an anion of tribasic acid such as $PO_4^{3-}$.

As another kinds of salts, salts of the carboxylate at the 4-position of the cephalosporin derivatives of formula (1), (1-1), (1-2), and (1-3) for instance, may be mentioned.

Examples of such a salt include an alkali metal salt (e.g., sodium salt, potassium salt, etc.); an magnesium salt, etc.); an ammonium salt; and an organic base salt (e.g., trimethylamine salt, triethylamine salt, pyridine salt, etc.).

The cephalosporin derivatives of formula (1), (1-1), (1-2), and (1-3) may have an esterified carboxyl group at the 4-position. As such esters, those which can be easily hydrolyzed physiologically are preferable.

Suitable examples of the ester may include alkoxyalkyl ester such as methoxymethyl, ethoxylmethyl, methoxyethyl and ethoxyethyl ester; alkanoyloxyalkyl ester such as acetoxymethyl, propionyloxymethyl, butyryloxymethyl, valeryloxymethyl and pivaloyloxymethyl ester; indanyl; phthalidyl; glycyloxymethyl; and phenylglycycloxymethy.

A cephalosporin derivative or its slat or ester thereof of the present invention is chemically stable in the form of crystal. Accordinly the crystalline cephalosporin derivative or its salt or ester thereof

is preferable when used as the active ingredient in the pharmaceutical composition. The crystal may be in the form of an anhydride or a hydrate such as 3/4·hydrate, 1·hydrate, 1,5·hydrate, 3·hydrate and 5·hydrate.

Specific examples of the cephalosporin derivative of the present invention are shown below.

Cephalosporin derivative of formula (1-1)

(100) (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate

(102) (6R,7R)-[(Z)-2-(2-aminothiazol-4-yℓ)-2-methoxyiminoacetoamido]-3-(1-azabicyclo[4,3,0]nonane-1-io)methyl-3-cephem-4-carboxylate

(104) (6R,7R)-[(Z)-2-(2-aminothiazol-4-yℓ)-2-methoxyiminoacetoamido]-3-(1-azabicyclo[4,4,0]decane-1-io) methyl-3-cephem-4-carboxylate

(106) (6R,7R)-7-[(Z)-2-(2-aminothiazol-4-yℓ)-2-methoxyiminoacetoamido]-3-(1-aza-8-methl-bicyclo[3,3,0]octane-1-io)methyl-3-cephem-4-carboxylate

(108) (6R,7R)-7-[(Z)-2-(2-aminothiazol-4-yℓ)-2-methoxyiminoacetoamido]-3-(1-aza-8-cyano bicyclo[3,3,0]octane-1-io)methyl-3-cephem-4-carboxylate

(110) (6R,7R)-7-[(Z)-2-(2-aminothiazol-4-yℓ)-2-methoxyiminoacetoamido]-3-(1-aza-8-hydroxymethylbicyclo[3,3,0]octane-1-io)methyl-3-cephem-4-carboxylate

(112) (6R,7R)-7-[(Z)-2-(2-aminothiazol-4-yℓ)-2-methoxyiminoacetoamido]-3-(1-aza-8-carbamoyl-

bicyclo[3,3,0]octane-1-io)methyl-3-cephem -4-carboxylate

(114) (6R,7R)-7-[(Z)-2-(2-aminothiazol-4-yℓ)-2-methoxyiminoacetoamido]-3-(1-aza-8-carbamoyloxybicyclo[3,3,0]octane-1-io)methyl-3-cephem -4-carboxylate

(116) (6R,7R)-7-[(Z)-2-(2-aminothiazol-4-yℓ)-2-(2-carboxypropyl-2-yloxyimino)acetoamido]-3-(1-azabicyclo[3,3,0]octane-1-io)methyl-3-cephem-4-carboxylate

(118) (6R,7R)-7-[(Z)-2-(2-aminothiazol-4-yℓ)-2-(5-methylimidazol-4-ylmethoxyimino)acetoamido] -3-(1-azabicyclo[3,3,0]octane-1-io)methyl -3-cephem-4-carboxylate

(120) (6R,7R)-7-[(Z)-2-(2-aminothiazol-4-yℓ)-2-carboxymethoxyiminoacetoamido]-3-(1-azabicyclo [3,3,0]octane-1-io)methyl-3-cephem-4-carboxylate

(122) (6R,7R)-7-[(Z)-2-(2-aminothiazol-4-yℓ)-2-(2-carboxyethoxyimino)acetoamido]-3-(1-azabicyclo[3,3,0]octane-1-io)methyl-3-cephem -4-carboxylate

(124) (6R,7R) -7-[(Z)-2-(2-aminothiazol-4-yℓ)-2-(imidazol-4-ylmethoxyimino)acetoamido]-3-(1-azabicyclo[3,3,0]octane-1-io)methyl-3-cephem-4-carboxylate

(126) (6R,7R)-7-[(Z)-2-(2-aminothiazol-4-yℓ)-2-(2-aminothiazol-4-yℓ-methoxyimino)acetoamido] -3-(1-azabicyclo[3,3,0]octane-1-io)methyl -3-cephem-4-carboxylate

(128) (6R,7R)-7-[(Z)-2-(2-aminothiazol-4-yℓ)-2-(1H-tetrazol-5-yl methoxyimino)acetoamido] -3-(1-azabicyclo[3,3,0]octane-1-io)methyl -3-cephem-4-carboxylate

(130) (6R,7R)-7-[(Z)-2-(2-aminothiazol-4-yℓ)-2-(1-methylimidazol-5-ylmethoxyimino)acetoamido] -3-(1-azabicyclo[3,3,0]octane-1-io)methyl-3-cephem-4-carboxylate

(132) (6R,7R)-7-[(Z)-2-(2-aminothiazol-4-yℓ)-2-(pyrolidone-3-ylmethoxyimino)acetoamido]-3-(1-azabicyclo[3,3,0]octane-1-io)methyl-3-cephem-4-carboxylate

(134) (6R,7R)-[(Z)-2-(2-aminothiazol-4-yℓ)-2-(2-carboxypropyl-2-yloxyimino)acetoamido]-3-(1-azabicyclo[4,3,0]nonane-1-io)methyl-3-cephem-4-carboxylate

(136) (6R,7R)-[(Z)-2-(2-aminothiazol-4-yℓ)-2-(2-carboxypropyl-2-yloxyimino)acetoamido]-3-(1-azabicyclo[4,4,0]decane-1-io)methyl-3-cephem-4-carboxylate

(138) (6R,7R)-[(Z)-2-(2-aminothiazol-4-yℓ)-2-(2-carboxypropyl-2-yloxyimino)acetoamido]-3-(1-aza-8-hydroxymethyl[3,3,0]octane-1-io)methyl-3-cephem-4-carboxylate

<u>Cephalosporin derivative of formula(1-2)</u>

(200) (6R,7R)-7-[(Z)-2-(2-aminothiazol-4-yℓ)-2-methoxyiminoacetoamido]-3-(1-azabicyclo[2,2,1]heptane-1-io)methyl-3-cephem-4-carboxylate

(202) (6R,7R)-7-[(Z)-2-(2-aminothiazol-4-yℓ)-2-(2-carboxy-propyl-2-yℓ-oxyimino)acetoamido]-3-(1-azabicyclo[2,2,1]heptane-1-io)methyl-3-cephem-4-carboxylate

(204) (6R,7R)-7-[(Z)-2-(2-aminothiazol-4-yℓ)-2-carboxymethoxyiminoacetoamido]-3-(1-azabicyclo[2,2,1]heptane-1-io)methyl-3-cephem-4-carboxylate

(206) (6R,7R)-7-[(Z)-2-(2-aminothiazol-4-yℓ)-2-(2-carboxyethoxyimino)acetoamido]-3-(1-azabicyclo[2,2,1]heptane-1-io)methyl-3-cephem-4-carboxylate

(208) (6R,7R)-7-[(Z)-2-(2-aminothiazol-4-yℓ)-2-

(imidazol-4-ylmethoxyiminoacetoamido]-3-(1-azabicyclo[2,2,1]heptane-1-io)methyl-3-cephem-4-carboxylate

(210) (6R,7R)-7-[(Z)-2-(2-aminothaizol-4-yℓ)-2-(5-methylimidazol-4-ylmethoxyimino)acetoamido]-3-(1-azabicyclo[2,2,1]heptane-1-io)methyl-3-cephem-4-carboxylate

(212) (6R,7R)-7-[(Z)-2-(2-aminothiazol-4-yℓ)-2-methoxyiminoacetoamido]-3-(1-aza-4-methyl-bicyclo[2,2,1]heptane-1-io)methyl-3-cephem 4-carboxylate

(214) (6R,7R)-7-[(Z)-2-(2-aminothiazol-4-yℓ)-2-methoxyiminoacetoamido]-3-(1-aza-4-hydroxymethylbicyclo[2,2,1]heptane-1-io)methyl-3-cephem-4-carboxylate

(216) (6R,7R)-7-[(Z)-2-(2-aminothiazol-4-yℓ)-2-methoxyiminoacetoamido]-3-(1-aza-4-chlormethylbicyclo[2,2,1]heptane-1-io)methyl-3-cephem-4-carboxylate

Cephalosporin derivative of formula (1-3)

(300) (6R,7R)-7-[(Z)-2-(2-aminothiazol-4-yℓ)-2-methoxyiminoacetoamido]-3-(1-aza-3-carbonyl-bicyclo[2,2,2]octane-1-io)methyl-3-cephem-4-carboxylate

(302) (6R,7R)-7-[(Z)-2-(2-aminothiazol-4-yℓ)-2-methoxyiminoacetoamido]-3-(1-aza-3-hydroxybicyclo[2,2,2]octane-1-io)methyl-3-cephem-4-carboxylate

(304) (6R,7R)-7-[(Z)-2-(2-aminothiazol-4-yℓ)-2-methoxyiminoacetoamido]-3-(1-aza-3-chloro-bicyclo[2,2,2]octane-1-io)methyl-3-cephem-4-carboxylate

(306) (6R,7R)-7-[(Z)-2-(2-aminothiazol-4-yℓ)-2-methoxyiminoacetoamido]-3-(1-aza-3-amino bicyclo [2,2,2]octane-1-io)methyl-3-cephem -4-carboxylate

(308) (6R,7R)-7-[(Z)-2-(2-aminothiazol-4-yℓ)-2-carboxypropyl-2-yloxyimino)acetoamido]-3-(1-aza-3-oxobicyclo[2,2,2]octane-1-io)methyl-3-cephem-4-carboxylate

(310) (6R,7R)-7-[(Z)-2-(2-aminothiazol-4-yℓ)-2-(2-carboxyethoxyimino)acetoamido]-3-(1-aza -3-oxobicyclo[2,2,2]octane-1-io)methyl-3-cephem-4-carboxylate

(312) (6R,7R)-7-[(Z)-2-(2-aminothiazol-4-yℓ)-2-(imidazol-4-yloxyimido)acetoamido]-3-(1-aza-3-oxobicyclo[2,2,2]octane-1-io)methyl-3-cephem-4-carboxylate

(314) (6R,7R)-7-[(Z)-2-(2-aminothiazol-4-yℓ) -2-(5-methylimidazol-4-ylmethoxyimino acetoamido]-3-(1-aza-3-oxobicyclo[2,2,2]-octane-1-io)methyl-3-cephem-4-carboxylate

(316) (6R,7R)-7-[(Z)-2-(2-aminothiazol-4-yℓ)-2-methoxyiminoacetoamido]-3-(1-aza-4-methyl bicyclo[2,2,2]octane-1-io)methyl-3-cephem -4-carboxylate

(318) (6R,7R)-7-[(Z) -2- (2-aminothiazol-4-yℓ) -2-methoxyiminoacetoamido]-3-(1-aza-4-cyanobicyclo[2,2,2]octane-1-io)methyl-3 -cephem-4-carboxylate

(320) (6R,7R)-7-[(Z)-2-(2-aminothiazol-4-yℓ)-2-methoxyiminoacetoamido]-3-(1-aza-4-ethoxy-carbonylbicyclo[2,2,2]octane-1-io)methyl -3-cephem-4-carboxylate

(322) (6R,7R)-7-[(Z)-2-(2-aminothiazol-4-yℓ)-2-methoxyiminoacetoamido]-3-(1-aza-4-hydro

xymethylbicyclo[2,2,2]octane-1-io)methyl-3-cephem-4-carboxylate

(324) (6R,7R)-7-[(Z)-2-(2-aminothiazol-4-yℓ)-2-methoxyiminoacetoamido]-3-[1-aza-4-(2,5-dihydro-2-methyl-5-oxotriazin-3-yℓ)thiomethylbicyclo[2,2,2]octane-1-io]methyl-3-cephem-4-carboxylate

The process for producing the cephalosporin derivative or its salt or ester thereof of this invention is described below in detail.

The cephalosporin derivative or its salt of ester thereof of this invention can be produced according to various methos (Reaction Schemes A to E) mentioned below, the details of which are explained in the following.

Reaction Scheme A

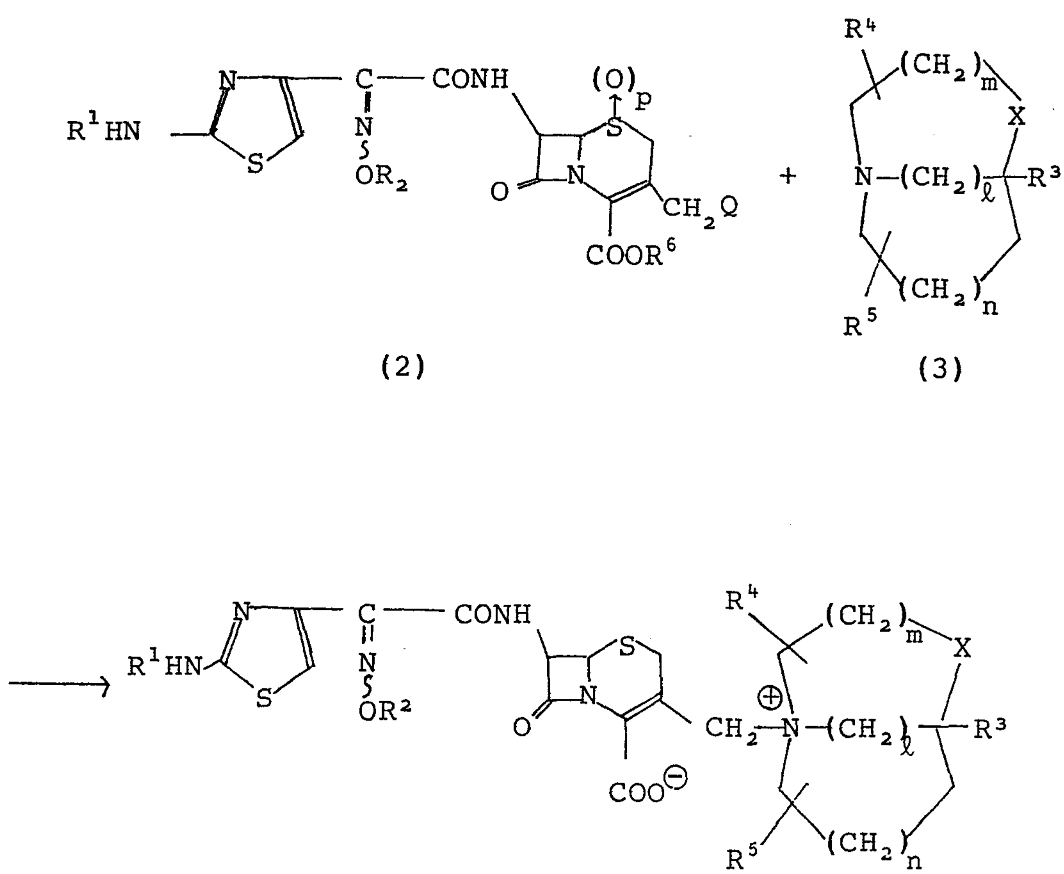

(2) (3)

(1)

In Reaction Scheme A, a cephalosporin compound of the formula (2) or its salt is made to react with a compound of the formula (3) or its salt. The reaction product, if required, is then subjected to deprotection, salt-forming reaction, esterification, or reduction.

In the formula(2), $R^1$ and $R^2$ are as defined in the formula (1), Q represents an acyloxy group, a carbamoyloxy group, or a halogen atom, p represents 0 or 1, and $R^6$ represents a hydrogen atom or a protective group.

Examples of preferred acyloxy groups include acetyloxy, trifluoroacetyloxy, trichloroacetyloxy, propionyloxy, 3-oxobutyryloxy, 3-carboxypropionyloxy, 2-carboxybenzoyloxy, 4-carboxybutyryloxy, mandelyloxy, 2-(carboethoxycarbamoyl) benzoyloxy, 2-(carboethoxysulfamoyl)benzoyloxy and 3-ethoxycarbamoylpropionyloxy.

Examples of preferred halogen atoms include iodine, bromine and chlorine.

Suitable exmples of the protective group in $R^6$ include lower alkyl such as methyl, ethyl, propyl, iso-propyl, butyl, pentyl and hexyl; alkanoyloxyalkyl such as acetoxymethyl, propionyloxymethyl, butyryloxymethyl, valeryloxymethyl and pivaloyloxymethyl; alkoxyalkyl such as methoxymethyl, ethoxymethyl, methoxyethyl and ethoxyethyl; aralkyl, which may be substituted, such as benzyl, 4-methoxybenzyl, 4-nitrobenzyl, phenetyl, trityl, diphenylmethyl, bis(methoxyphenyl)methyl and 3,4-dimethoxybenzyl; haloalkyl such as 2-iodoethyl and 2,2,2-trichloroethyl; alkenyl such as vinyl and allyl; aryl which may be substituted such as phenyl, 4-chlorophenyl, tolyl, xylyl and mesityl; and trialkylsilyl such as trimethylsilyl, triethylsilyl, tert-butyldimethylsilyl and tributylsilyl.

Of these, $R^6$ is preferably the trialkylsilyl group. In case where $R^6$ is the trialkylsilyl group, the cephalosporin derivative (1) can be obtained in good yield because the production of $\Delta^2$-isomer of the cephalosporin derivative (1) is inhibited.

The cephalosporin compound of the formula (2) may be its salt, and suitable examples of the salt include the same salts that are exemplified in the cephalosporin derivatives of the formula (1), (1-1), (1-2) and (1-3).

The cephalosporin compound of the formula (2) is a known compound and can be produced by known methods (U.S.Patent No.4,298,606, U.S.Patent No. 4,406,899, and U.S.Patent No. 4,399,131.)

In the formula (3), $R^3$, $R^4$, $R^5$, $\ell$, m, n and X are as defined in the formula (1). The compound of the formula (3) may be its salt, and suitable salts may include an acid addition salt such as an inorganic acid salt (e.g., hydrochloride, hydrobromide, sulfate, phosphate, etc.); and an organic acid salt (e.g., acetate, maleate, tartrate, benzensulfonate, toluenesulfonate, etc.).

The compound of the formula (3) is a known compound and can be produced by known methods (J. Org. Chem. 46, 1737-1738, 1981; Synthesis, 701, September, 1978).

The reaction of a cephalosporin compound of the formula (2) or its salt with a compound of the formula (3) or its salt is effected by allowing them to come into contact with each other in an inert organic solvent.

Examples of the inert organic solvent include halogenated hydrocarbons such as methylenechloride, dichloromethane, chloroform, carbontetrachloride and 1,2-dichloroethane; ethers such as diethyl ether, tetrahydrofuran, dioxane and dimethoxyethane; hydrocarbons such as hexane, benzene, toluene and xylene;

acetonitrile; dimethylformamide; diethylacetoamide; dimethylsulfoxide; and ethylacetate.

It is desirable to maintain the reaction temperature at or lower than room temperature, and it is advisable to carry out the reaction at a temperature ranging from -30° to +50°C.

The reaction of the cephalosporin compound of the formula (2) or its salt with the compound of the formula (3) or its salt is theoretically accomplished in equimolecular quantities; however, in the actual reaction the compound of formula (3) or its salt is usually used 0.7 to 10 times, preferably 1.0 to 10 times the molar quantity of the cephalsporin compound of formula (2) or its salt.

The reaction can be carried out by simply stirring them together. The reaction time varies depending upon the reaction solvent and reaction temperature, but usually ranges from 5 minutes to 3 hours.

The reaction product can be isolated and purified by known methods such as crystallization, chromatography, etc.

In case where a syn isomer of the compound (2) or its salt is used as the material compound in the reaction, a syn isomer of the compound (1) or its salt or ester thereof is obtained. When syn and anti isomers of the compound (2) or salt thereof are used, a mixture of syn and anti isomers of the compound (1) is obtained. The mixture can be separated into the respective isomers according to the usual method such as crystallization and chromatography.

The reaction product is, if required, subjected to deprotection, salt-forming reaction, esterification or reduction.

The deprotection is a known reaction <u>per se</u> and

a carboxyl-protecting group can be released by the hydrolytic cleavages in the presence of acid or base. An aminoprotecting group can be removed by contact with acid or by catalytic reduction (U.S.Patent No. 4,152,433; U.S.Patent No.4,298,606).

The salt-forming reaction is also a known reaction *per se* and the reaction can be effected, for instance, by treating a compound of formula (1) having a betaine structure with an acid such as hydrochloric acid, hydrobromic acid; sulfuric acid, nitric acid, phosphoric acid, acetic acid, and trifluoroacetic acid; or otherwise treating betaine with a salt such as sodium chloride, sodium iodine, and potassium chloride in the presence of an acid.

The esterification is a known reaction itself and is carried out by allowing a cephalosporin derivative of the formula (1) or its salt to react with alkylhalide, alkanoyloxyalkylhalide or alkoxyalkylhalide, for instance, in an inert organic solvent such as acetone and dimethylformamide (United Kingdom Patent No.2,404,921).

The reduction is also a known reaction itself and is accomplished by treating a cephalosporin derivative of the formula (1) wherein p is 1 with acetylchloride or the like and further by reducing with sodium dithionous acid (United Kingdom Patent No.2,40,921).

The following methods may be cited for obtaining the crystalline cephalosporine derivatives of the present invention.

There are a method in which an amorphous powder of cephalosporin derivative of formula (1) is firstly dissolved in water, or an organic solvent such as methanol, ethanol, acetone, tetrahydrofuran, dioxane, and acetonitrile, or a mixed solvent thereof and

then allowing the solution standing awhile until the crystal is formed and precipitated; a method in which said amorphous powder is recrystallized by use of the abovementioned solvents; and a method in which said amorphous powder is first dissolved in water and then the aforementioned organic solvent is added to the solution to have the resulting crystal precipitated.

On the other hand, there is another method in which an acid addition salt of the cepholosporin derivative (1) is dissolved in water or an organic solvent such as methanol, ethanol and propanol or a mixed solvent thereof and then nautralized with a base such as triethylamine, sodium hydroxide and sodium carbonate to yield crystal.

In the abovementioned crystallization or recrystallization effected in water or aqueous solution, a crystalline hydrate is usually obtained. In case where crystallization is carried out in an organic solvent, a crystalline anhydride or solvate is obtained. The solvate can be easily converted into an anhydride. The crystalline anhydride or solvate can be converted into a hydrate by allowing it in a gas which contains water vapor.

Reaction Scheme B

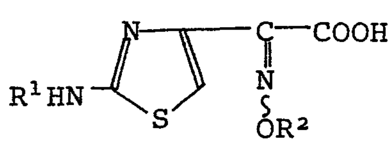

(4)

+

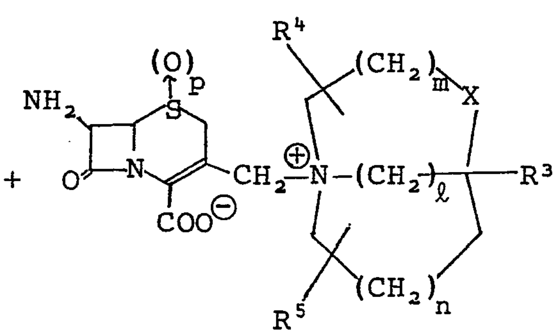

(5)

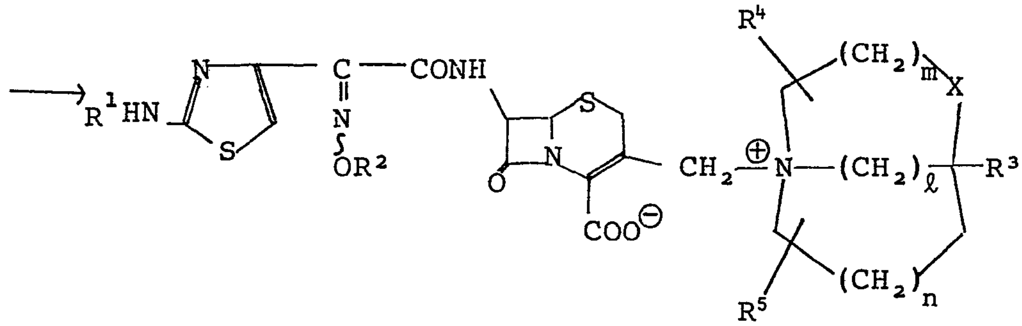

(1)

In Reaction Scheme B, the compound of the formula (4) or its salt or reactive derivative thereof is made to react with the compound of the formula (5) or its salt or ester thereof or reactive derivative thereof. The reaction product, if required, is then subjected to deprotection, salt-forming reaction, esterification, or reduction.

In the formula (4), $R^1$ and $R^2$ are as defined hereinabove.

The salt of the compound (4) may include an acid addition salt. Examples of the acid addition salt are the same as exemplified in the compound (3) in Reaction Scheme A.

As the reactive derivative of the compound (4), there are acid halide, acid anhydride, mixed acid anhydride, active amide and active ester.

Examples of acid halide are acid chloride and acid bromide. Examples of mixed acid anhydride are mixed acid anhydrides with an acid such as dialkylphosphoric acid, phenylphosphoric acid, pivalic acid and pentanoic acid. Examples of active amide are activated amide with imidazol, triazole, tetrazol or dimethylpyrazole. Examples of active ester are cyanomethyl ester, methoxymethyl ester, dimethyl-

iminomethyl ester, p-nitrophenyl ester and mesyl phenyl ester.

The compound (4) is a known compound and can be produced by a known method (U.S.Patent No. 4,298,606).

In the formula (5), $R^3$, $R^4$, $R^5$, $\ell$, m, n and p are as defined above. As the salt of the compound (5), there may be employed an alkali metal salt such as sodium salt and potassium salt; an alkaline earth metal salt such as calcium salt and magnesium; an ammonium salt; and an organic base salt such as trimethylamine salt, triethylamine salt and pyridine salt.

Examples of esters of the compound (5) may be the compounds esterified with the same protective group as exemplified for $R^6$ of the compound (2) in Reaction Scheme A.

Suitable reactive derivatives of the compound (5) may include Schiff's base type imino or its tautomeric enamine type isomer formed by the reaction with a carbonyl compound such as acetoacetic acid; a silyl derivative formed by the reaction with a silyl compound such as bis(trimethylsilyl)acetamide, trimethylchlorosilane and tert-butyldimethylsilane; a derivative formed by the reaction with phosphorus trichloride or phosgene.

The compound (5) can be produced according to the following method.

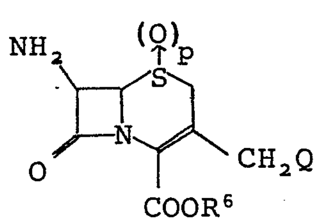

(5')

+

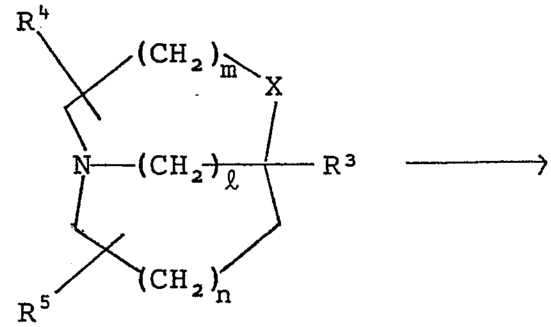

(3)

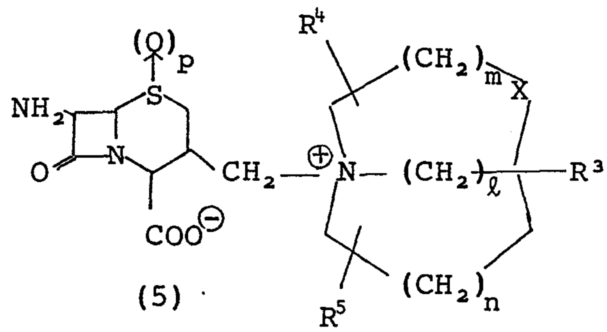

(5)

The above reaction can be carried out according to the same way as the reaction of the compound (2) with the compound (3) shown in Reaction Scheme A.

The reaction between the compound (4) or its salt or reactive derivative thereof and the compound (5) or its salt or ester thereof or reactive derivative thereof is effected by allowing them come into contact with each other in an inert organice solvent and, if necessary, in the presence of a condensing agent or a base.

Examples of the inert organic solvent include halogenated hydrocarbons such as methylenechloride dichloromethane, chloroform, carbontetrachloride and 1,2-dichloroethane; ethers such as diethyl ether, tetrahydrofuran, dioxane and dimethoxyethane; hydrocarbons such as hexane, benzene, toluene and xylene; acetonitrile; dimethylformamide; diethylacetoamide; and dimethylsulfoxide.

In carrying out the reaction, a base may be added to the reaction system. Examples of the base include organic or inorganic salts of alkali metal hydroxides, alkali metal hydrogencarbonates, alkali metal carbonates, trialkylamine, N,N-dialkybenzylamine, pyridine, and alkylmorpholine. The amount of the base to be used in the reaction is 1 to 3

times the molar quantity of the compound (4) or its salt or its reactive derivative.

In case where the compound (4) or its salt is used in the reaction, a condensing agent may be added to the reaction system. Examples of the condensing agent may N,N-dicyclohexylcarbodiimide, N-cyclohexyl-N'-morpholinoethylcarbodiimide, N,N'-diethylcarbodiimide, trimethyl phosphite, triphenylphosphine and 2-ethyl-7-hydroxybenzisoxazolium salt. The amount of the bases to be used is usually 1 to 3 times the molar quantity of the compound (4) or its salt. The reaction is usually effected with the use of approximately equimolar amount of the compound (4) or its salt or reactive derivative thereof and the compound (5) or its salt or ester thereof or reactive derivative thereof.

The reaction is carried out with cooling or at room temperature.

The reaction is usually completed in several minutes to several hours.

The reaction product can be isolated and purified according to known methods such as extraction with a solvent, crystallization, and chromatography.

The deprotection, salt-forming reaction, esterification, or reduction can be conducted in the same way as Reaction Scheme A.

The desired compound in the crystal form can also be obtained in the same way as described in Reaction Scheme A.

In case where a syn isomer of the compound (4) or its salt or reactive derivative thereof is used as the material compound in the reaction, a syn isomer of the compound (1) or its salt or ester thereof is obtained. When syn and anti isomers of the compound (4) or salt thereof or reactive derivative

thereof are used, a mixture of syn and anti isomers of the compound (1) is obtained. The mixture can be separated into the respective isomers according to usual method such as crystallization and chromatography.

Reaction Scheme C

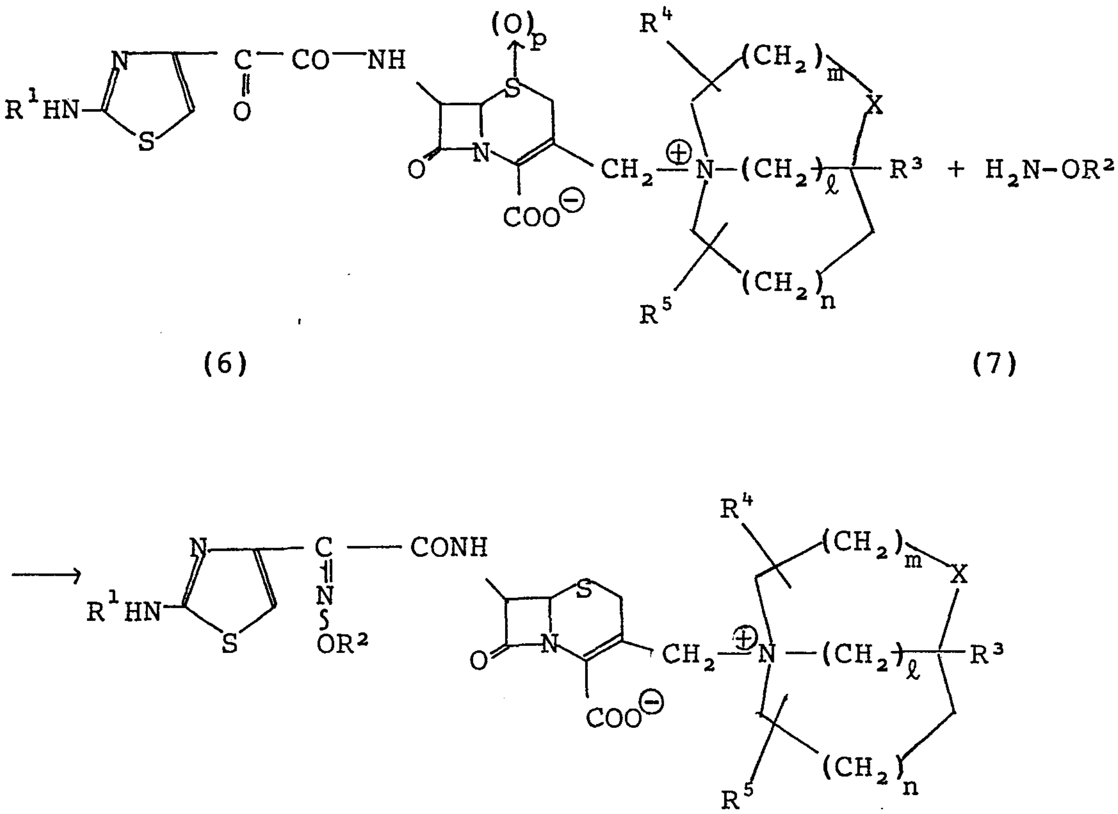

+ $H_2N-OR^2$

(6) (7)

(1)

In Rection Scheme C, the compound (6) or its salt or ester thereof is made to react with the compound (7) or its protected compound or salt thereof. The reaction product, if required, is then subjected to deprotection, salt-forming reaction, esterification or reduction.

In the formula (6), $R^1$, $R^3$, $R^4$, $R^5$, $\ell$, m, n

and p are as defined hereinbefore.

As the salt of the compound (6), there are acid addition salts, alkali metal salts, alkali earth metal salts, ammonium salt, and organic base salts. As the examples of these salts, those salts as described with regard to the compound (1) may be mentioned again.

Examples of ester of the compound (6) may be the compounds esterified with the same protective group as exemplified with regard to $R^6$ of the compound (2) in Reaction Scheme A.

The compound (6) can be produced according to the following method.

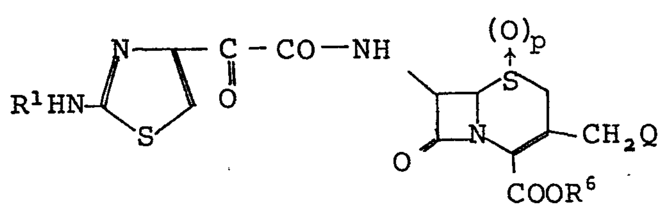

+

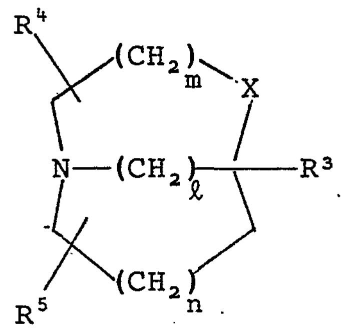

(6') (3)

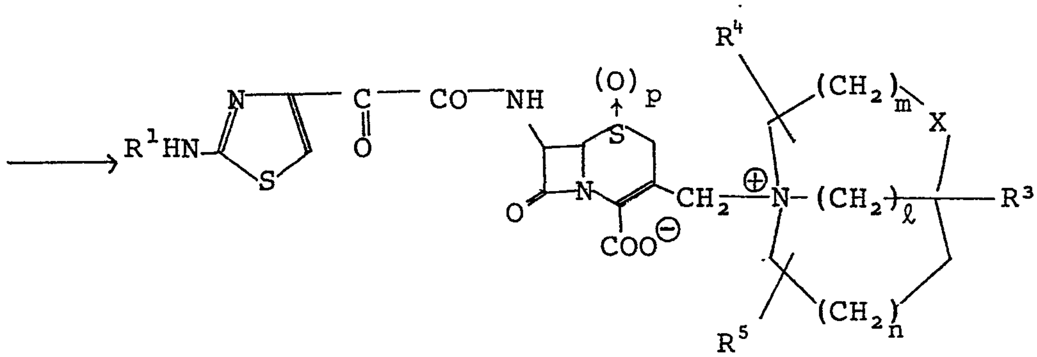

(6)

The compound (6') is a known compound and can be produced by the known method (U.S.Patent No. 4,201,779; U.S.Patent No. 4,288,436; U.S.Patent No. 4,411,898)

The above reaction can be carried out according to the same way as the reaction of the compound (2) with the compound (3) described in Reaction Shceme A.

In the formula (7), $R^2$ is as defined hereinbefore. As the protected compound of the compound (7), in which $R^2$ has an active substituent such as a carboxyl group, such compounds as those whose substituent is protected by a conventional protective group may be mentioned.

As the salt of the compound (7), an acid addition salt is used and examples of the salt are as same as those mentioned before.

The compound (7) can be produced by a known method using hydroxyphthalimide (Bulletin Society, 833, 1976).

The reaction between the compound (6) or its salt or ester thereof and the compound (7) or its protected compound or salt thereof is effected by allowing them to come into contact with each other in an inert organic solvent and, if necessary, in the presence of a base.

Examples of the inert organic solvent include alcohols such as methanol, ethanol, propanol and butanol. These inert organic solvents may contain water.

As the base to be used in case of necessity, the same bases that are used in Reaction Scheme B may be mentioned.

The amounts of such bases to be used in the reaction is usually 1 to 3 moles to 1 mole of the compound (6) or its salt or ester thereof. The reaction of the compound (6) or its salt or ester

thereof with the compound (7) or its protected compound or salt thereof is usually effected in equimolecular amounts.

The reaction is usually carried out with cooling or at room temperature.

The reaction time usually ranges from several minutes to several hours. The isolation and purification of the reaction product can be effected by any known methods such as extraction by solvent, crystallization and chromatography.

In the present reaction, a mixture of syn and anti isomers of the cephalosporin derivative (1) is obtained. The mixture can be separated into the respective isomer by usual method such as recrystallization and chromatography.

The deprotection, salt-forming reaction, esterification, or reduction can be conducted in the same way as in the case of Reaction Scheme A. The desired compound can also be obtained in the form of a crystal in the way similar to the one adopted in Reaction Scheme A.

Reaction Scheme D

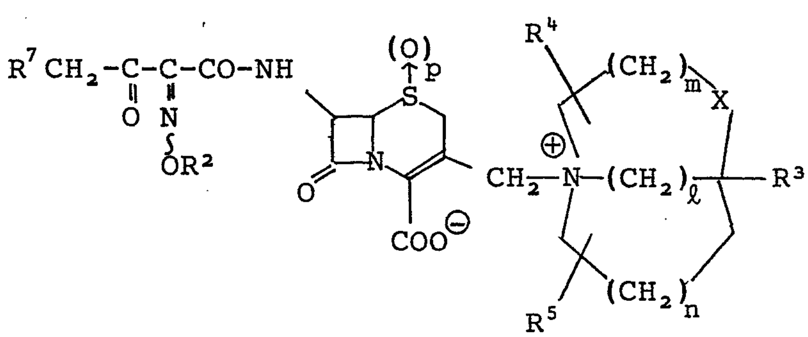

+

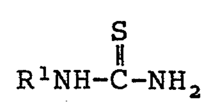

(8) (9)

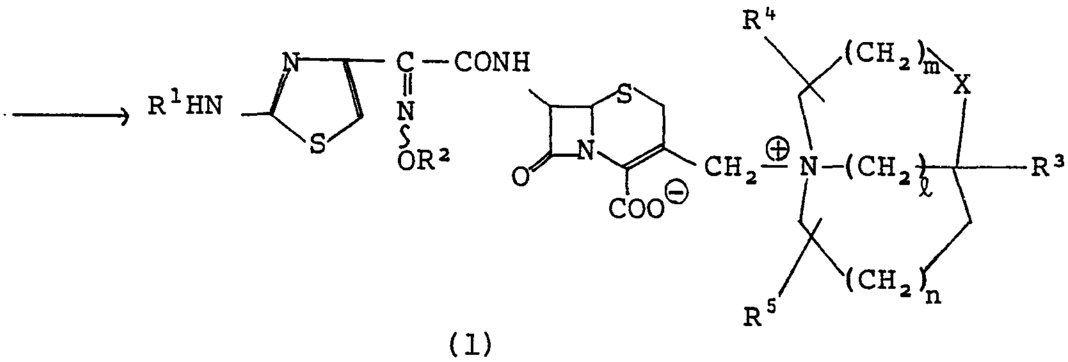

(1)

In Reaction Scheme D, the compound (8) or its salt or ester thereof is made to react with the compound (9) or its salt. The reaction product, if required, is then subjected to deprotection, salt-forming reaction, esterification or reduction.

In the formula (8), $R^2$, $R^3$, $R^4$, $R^5$, X, ℓ, m, n and p are as defined hereinbefore, and $R^7$ represents a halogen atom such as chlorine, bromine and iodine.

As the salt of the compound (8), there are, for instance, alkali metal salts, alkali earth metal salts, ammonium salt, and organic base salts. To show the example of such salts, the salts similar to those described with regard to the compound (1) may be mentioned.

Examples of esters of the compound (8) may be the compounds esterified with the same protective group as exemplified for $R^6$ of the compound (2) described in Reaction Scheme A.

The compound (8) can be produced according to the following method.

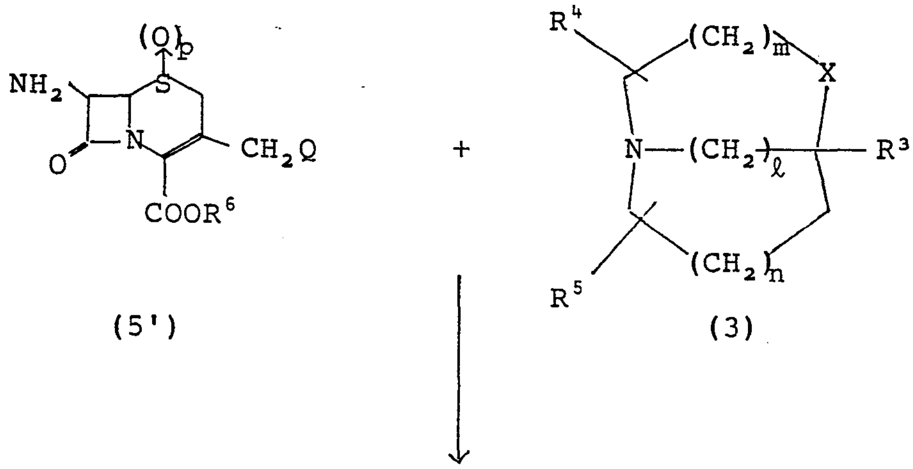

(5')

+

(3)

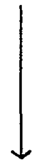

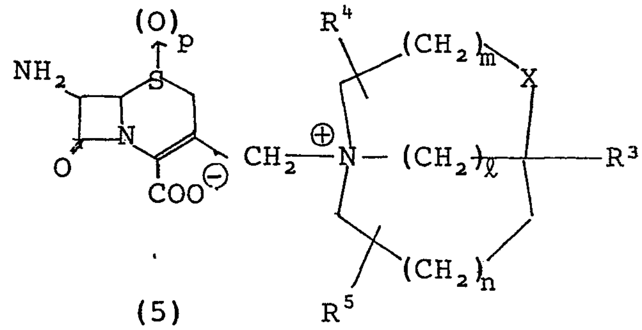

(5)

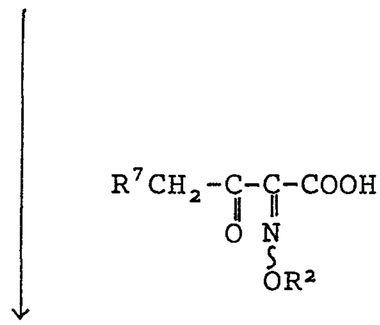

(8')

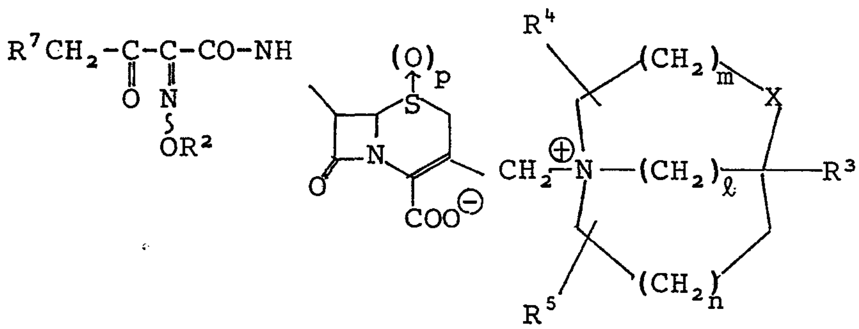

(8)

In the aforementioned reaction sheme, the reaction between the compound (5') and the compound (3) is accomplished following the same way described in Reaction Scheme A. The reaction between the compounds (5) and (8') is carried out according to the same method of the reation described in Reaction Sheme B. The compound (8') is a known compound and can be produced by the known method (United Kingdom Patent No. 2,012,276).

In the formula (9), $R^1$ represents a hydrogen atom or a protective group. The compound (9) is

a known compound (U.S.Patent No. 4,298,606). As the salt of the compound (9), an acid addition salt may be mentioned. The examples of the acid addition salt may include the same addition salts that are cited with regard to the compound (1).

The reaction of the compound (8) or its salt or ester thereof with the compound (9) or its salt is effected by allowing them to come into contact with each other in an inert solvent.

Examples of the inert solvent include water; alcohols such as methanol, ethanol, propanol, and butanol; ethers such as diethyl ether, tetrahydrofuran and dioxane; dimethylformamide; dimethylacetoamide; and methylpiperidone. A mixed solvent comprising any of these solvents is also usable.

The compound (9) or its salt is usually used 1 to 3 times the moler quantity of the compound (8) or its salt or ester thereof.

No specific limits are set to the reaction temperature; however, it is a usual practice to effect the reaction at a temperature ranging from a room temperature up to the boiling point of the solvent.

The reaction time is usually within the range between 1 to 10 hours.

The reaction product can be isolated and purified following known method such as solvent extraction, crystallization and chromatography.

In case where a syn isomer of the compound (8) is used as the material compound in the reaction, a syn isomer of the compound (1) is obtained. When syn and anti isomers of the compound (8) are used, a mixture of syn and anti isomers of the compound (1) is obtained. The mixture can be separated into the respective isomers according to the usual

method such as crystallization and chromatography.

The deprotection, salt-forming, esterification or reduction reaction can be conducted in the same way as Reaction Scheme A. The crystalline anhydride or hydrate of the desired compound can also be obtained in the same way as Reaction Scheme A.

Reaction Scheme E

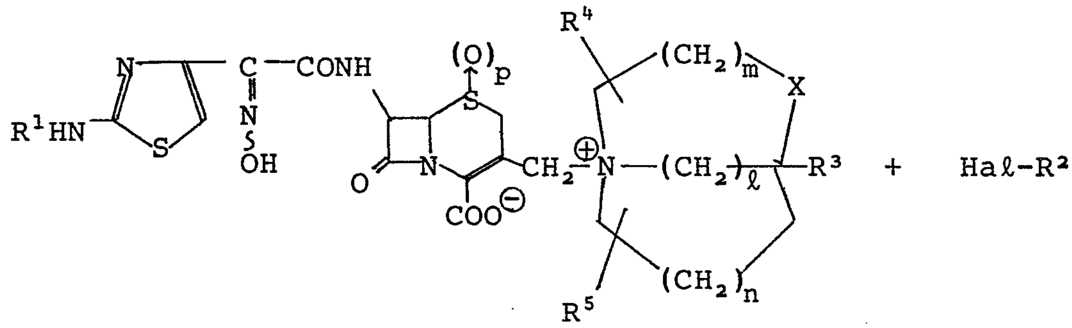

(1''') (10)

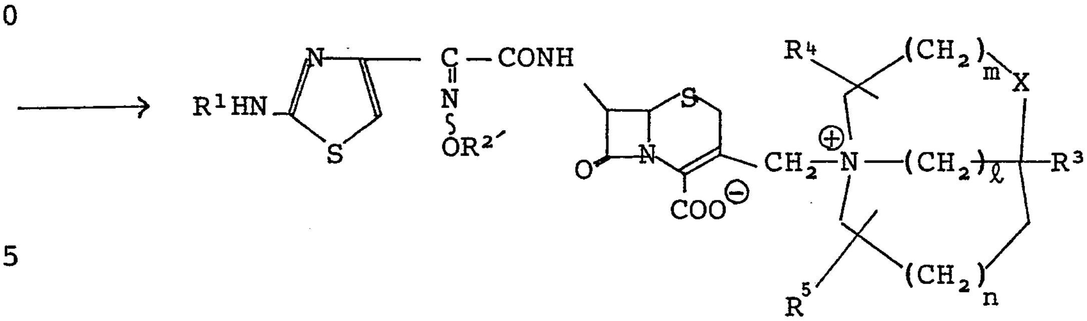

(1'''')

In Rection Scheme E, the compound (1''') or its salt or ester thereof is made to react with the compound (10), a dialkylsulfate or a diazoalkane. The reaction product, if required, is then subjected to deprotection, salt-forming reaction, esterification or reduction.

In the formula (1'''), $R^1$, $R^3$, $R^4$, $R^5$, X, $\ell$, m, n and p are as defined hereinbefore.

As a salt of the compound (1'''), there are acid addition salts, alkali metal salts, alkali earth metal salts, ammonium salt, and organic salt bases. As the example of these salts, the same salts that are mentioned with regard to the compound (1) may be mentioned.

Examples of ester of the compound (1''') may be the compounds esterified with the same protective group as exemplified for $R^6$ of the compound (2) in Reaction Scheme A.

The compound (1''') can be produced according to the following method.

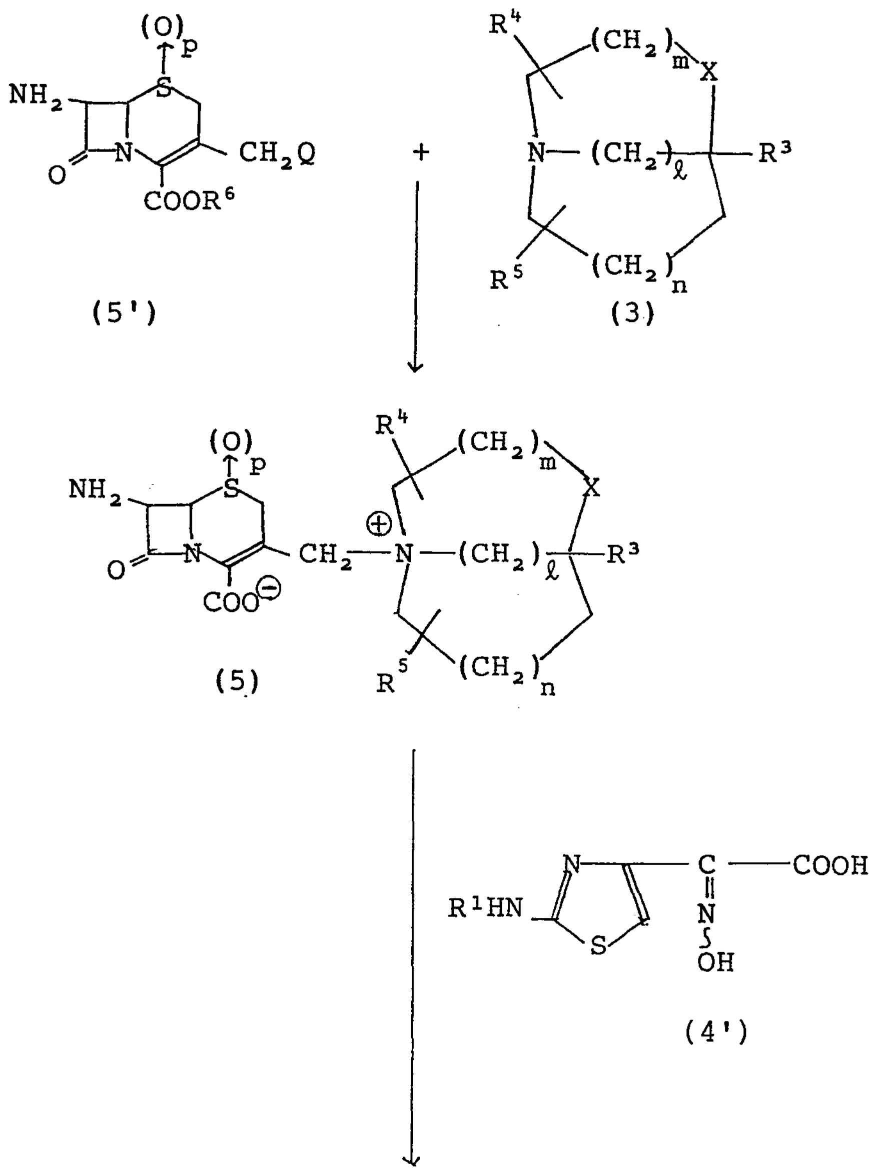

(5') (3)

(5)

(4')

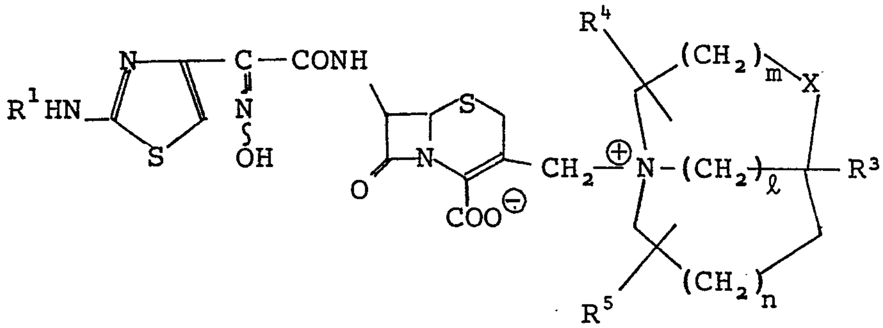

(1''')

In the abovementioned reaction scheme, the reaction between the compound (5') and the compound (3) can be effected in the same way as the one described in Reaction Scheme A and the reaction of the compound (5) with the compound (4') can be conducted in the same way as the one described in Reaction Scheme B. The compound (4') is a known compound and can be produced by the known method (U.S.Patent No. 4,298,606).

In the formula (10), $R^{2'}$ is as defined above. The compound (10) can be obtained by halogenating corresponding aliphatic hydrocarbon, cyclic hydrocarbon or heterocyclic compound according to the conventional method.

Examples of the dialkylsulfate, for instance may include dimethylsulfate and diethylsulfate. Examples of the diazoalkane, for instance, may include diazomethane.

The reaction between the compound (1''') or its salt or ester thereof with the compound (10), dialkylsulfate or diazoalkane is effected by allowing them to come into contact with each other in an inert solvent and, if necessary, in the presence of a base.

Examples of the inert solvent include alcohols

such as methanol, ethanol, propanol and butanol; halogenated hydrocarbons such as methylenechloride, dichloromethane, chloroform, carbontetrachloride and 1,2-dichloroethane; ethers such as diethyl ether, tetrahydrofuran, dioxane and dimethoxyethane; dimethylformamide; diethylacetoamide; ethylacetate; and water.

If necessary, the reaction may be carried out in the presence of a base. As such base, the organic or inorganic bases such as alkali metal hydroxide, alkalimetal hydrogencarbonate, alkali metal carbonate, trialkylamine, N,N-dialkylbenzylamine, pyridine, and N-alkylmorpholine may be mentioned. The amount of the base to be used in the reaction is usually 1 to 3 moles to 1 mole of the compound (1''') or its salt or ester thereof.

The compound (10), dialkylsulfate or diazoalkane is usually used in an amount of 1 to 3 moles against 1 mole of the compound (1''') or its salt or ester thereof.

Though no limit is specifically set upon the reaction temperature, the reaction is usually carried out at temperatures ranging from cooling up to heating at somewhere around the boiling point of the solvent.

The reaction time is usually in the range of several minutes to several days.

The isolation and purification of the reaction product can be achieved by the known method such as solvent extraction, crystallization and chromatography.

In case where a syn isomer of the compound (1''') is used as the material compound in the abovementioned reaction, a syn isomer of the compound (1'''') is obtained. In case where a mixture of syn and anti isomers of

the compound (1''') is used, a mixture of syn and anti isomers of the compound (1'''') is obtained. The obtained mixture can be separated according to the usual technique such as crystallization and chromatography.

The deprotection, salt-forming reaction, esterification, or reduction can be conducted in the same way as in Reaction Scheme A. The crystalline anhydride or hydrate of the desired compound can also be obtained in the same way that is described in Reaction Scheme A.

The cephalosporin derivative of formula (I) or its salt or ester thereof of the present invention, especially the cephalosporin derivative of formula (I), in which $R^1$ is a hydrogen atom, or its pharmaceutically acceptable salt or ester thereof, has a highly effective antibacterial activity against the wide varieties of Gram-positive and Gram-negative bacteria and is very useful for the treatment of human beings, including of animals, suffering from bacterial infectious diseases. The cephalosporin derivative of this invention displays a strong antibacterial action especially against Gram-negative such as Pseudomonas aeruginosa, Salmonera typhosa and Enterobacter or Gram-positive such as Staphyrococcus, Streptococcus and Butilus subtilus. Also, the cephalosporin derivative of this invention has a dominant characteristics of having a long duration time.

The cephalosporin derivative or its pharmaceutically acceptable salt or ester thereof proposed by this invention is administered parenterally or orally. The dosage form for parenteral administration includes, for instance, an intravenous or intramuscular injection and suppository. An injection may be prepared in the form of an aqueous by oily

solution and suspention. It may also be prepared as the dispensing powder of crystal to be made into an injection with sterile water at the time of its administration. The suppository may be prepared as the preparation with the use of ordinary vehicles and excipients such as cocoa butter, glyceride, etc. and, if necessary, absorbefacients.

For oral administration, there are ordinary capsules, tablets, and granules, which may be allowed to contain absorbefacient, preservative, etc.

The abovementioned preparations of various dosage forms can be manufactured according to the methods known to the persons skilled in the art.

The dosage of the cephalosporin derivative or its pharmacetuically acceptable salt or ester thereof of this invention varies depending upon the dosage regimen including the age of a patient, condition of a disease, etc.; however, it is usually in the range of 20 to 2000 mg/day.

The following examples illustrate the present invention more specifically.

Example 1

Synthesis of (6R,7R)-7-[(Z)-2-(2-aminothiazol-4-yℓ)-2-methoxyiminoacetoamido)-3-(1-azabicyclo[3,3,0]octane-1-io)methyl-3-cephem-4-carboxylate [compound(100)]

To a suspension of 468 mg of diphenylmethyl (6R,7R)-7-[(Z)-2-(2-tritylaminothiazol-4-yℓ)-2-methoxyimino acetoamido]-3-iodomethyl-3-cephem-4-carboxylate in 30 mℓ of ether was added 111 mg of 1-azabicyclo[3,3,0]octane and the mixture was thoroughly stirred at room temperature for 1.5 hours. The precipitate was passed through the filter and 3 mℓ of trifluoroacetic acid was added to the resulting cake (485 mg). The mixture was stirred at room temperature for 1.5 hours. Trifluoroacetic acid was then removed at a temperature below 20°C under reduced pressure and the obtained residue was pulverized in ether, collected by filtration and then dissolved in a small amount of methanol. The solution was passed through a column of HP-20 ion-exchange resin, and eluted with a mixed solvent of water and methanol while gradually increasing the volume percentage of methanol from 0% to 40%. The fractions eluted with 20%~40% ethanol in water were collected and lyophilized to obtain 47 mg of powder containing the compound (100).

IR ($cm^{-1}$):
1770, 1668, 1630, 1220, 1130, 1040

NMR (ppm, $D_6$ DMSO) δ:
1.7-2.3 (8H, broad)
3.2-4.0 (5H, broad)
3.85 (3H, s) 4.7-4.7 (4H, multiplet)
5.27 (1H, d) 5.89 (1H, d+d)
6.84 (1H, s) 7.26 (2H, $NH_2$)

Example 2

Synthesis of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate·sulfuric acid salt [sulfate of compound (100)]

(i) A solution was prepared by dissolving 3.0 g of (6R,7R)-7-[(Z)-2-(2-aminothiazol-4-yℓ)-2-methoxyimino-acetoamido]-3-(1-azabicyclo[3,3,0]octane-1-io) methyl-3-cephem-4-carboxylate [compound (100)] in 7 mℓ of water, to which 6 mℓ of 10% sulfuric acid was added dropwise with stirring and cooling with ice-cold water. About 15 mℓ of ethanol was added thereto and left standing until crystallization was completed. The precipitated crystal was filtered and the obtained crystal was washed with ethanol and dried to give 2.5 g of sulfate of compound (100) as crystals.

IR ($cm^{-1}$):
3370, 1788, 1640, 1545, 1070, 1030
NMR (ppm, $D_2O$) δ:
1.8-2.3 (8H broad)
3.2-3.8 (5H broad)
4.0 (3H singlet)
4.0-4.7 (4H broad)
5.3 (1H doublet)
5.8 (1H doublet)
7.05 (1H singlet)

(ii) 4.0 g of (6R,7R)-7-[(Z)-(2-aminothiazol-4-yℓ)-2-methoxyiminoacetoamido-3-(1-azabicycl0[3,3,0]octane-1-io)methyl-3-cephem-4-carboxylate·2trifluoro acetic acid salt [trifluoroacetate of compound (100)] was dissolved in 15 mℓ of water under heating and 8.5 mℓ of 10% sulfuric acid was then added thereto while cooling with ice-cold water. 5 mℓ of ethanol was added to the mixture and was kept at -20°C overnight to form a white crystal. The white crystal was filtered and dried to obtain 3.3 g of the sulfate of compound (100) as crystals.

Example 3

Synthesis of (6R,7R)-7-[(Z)-2-(2-aminothiazol-4-yℓ)-2-methoxyimino acetoamido]-3-(1-azabicyclo[3,3,0]octane-1-io)methyl-3-cephem-4-carboxylate·2hydrochloric acid salt[hydrochloride of compound (100)]

3.0 g of (6R,7R)-[(Z)-2-(2-aminothiazol-4-yℓ)-2-methoxyiminoacetoamido]-3-(1-azabicyclo[3,3,0]octane-1-io)methyl-3-cephem-4-carboxylate[compound(100)] was dissolved in a mixed solvent consisting of 7 mℓ of water and 5 mℓ of methanol. After 4 mℓ of 4N HCℓ was added little by little to the solution while cooling with ice-cold water, 20 mℓ of ethanol was added thereto. The mixture was kept cooling with ice overnight and the resulting crude crystal was recrystallized with water to give 2.2 g of the hydrochloride of compound (100) as crystals.

IR ($cm^{-1}$):
3390, 1770, 1709, 1650, 1036.

Example 4

Synthesis of (6R,7R)-7-[(Z)-2-(2-aminothiazol--4-yl)-2-methoxyiminoacetoamido]-3-(1-azabicyclo[3,3,0]octane-1-io)methyl-3-cephem-4-carboxylate in the form of crystal[crystalline compound (100)

(i) 5.0 g of (6R,7R)-7-[(Z)-2-(2-aminothiazol-4-yl)-2-methoxyiminoacetoamido]-3-(1-azabicyclo[3,3,0]octane-1-io)methyl-3-cephem-4-carboxylate [amorphous compound (100)] was dissolved in 20 ml of methanol at room temperature and the solution was stirred for 2 hours. The precipitated crystal was separated from the solution by filtration, washed with methanol, and dried to give 4.8 g of the final product as crystals.

The result of the X-ray diffraction measurement conducted for this product gave the following relative intensity which showed the presence of the crystal.

| Angle of diffraction (2θ°) | Relative intensity (%) |
|---|---|
| 6.41 | 35 |
| 7.28 | 72 |
| 13.46 | 36 |
| 13.88 | 27 |
| 14.29 | 20 |
| 17.85 | 100 |
| 19.02 | 40 |
| 20.45 | 30 |
| 22.40 | 95 |
| 23.23 | 21 |
| 23.01 | 22 |
| 23.97 | 24 |
| 25.80 | 28 |

| | |
|---|---|
| 26.43 | 26 |
| 28.22 | 19 |
| 28.75 | 25 |

The infrared absorption spectra showed the absorption in the following wavelengths different from amorphous:

3300, 3190, 1763, 1675, 1617, 1350, 1300, 1210, 1182, 1098, 1015, 961, 926, 889, 860, 823, 781, 762, 737, ($cm^{-1}$).

(ii) 6.0 g of (6R,7R)-7-[(Z)-2-(2-aminothiazol-4-yl)-2-methoxyiminoacetoamido]-3-(1-azabicyclo[3,3,0]octane-1-io)methyl-3-cephem-4-carboxylate·sulfuric acid salt [sulfate of compound (100)] was dispersed in 15 ml of methanol. Upon addition of 2.77 ml of triethylamine dropwise at room temperature, the dispersed compound gradually dissolved. After the 5 hours - stirring, the precipitated crystal was separated by filtration to obtain 4.9 g of the final product as crystals.

The X-ray diffraction and infrared absorption spectrum of this crystal coincided with those of the crystal obtained in the preceding (i).

(iii) 4.8 g of the crystal was prepared following the procedure of (ii) except for the use of 5.8 g of hydrochloride of compound (100) in the place of sulfate of compound (100) used in (ii).

(iv) Also 4.9 g of the crystal was obtained following the procedure of (ii) except for the use of 7.3 g of trifluoroacetate of compound (100) in the place of sulfate of compound (100) used in (ii).

(v) The crystal obtained in (ii) was allowed to stand in air(relative humidity:50~60%) overnight to give a 3·hydrate of the crystal.

The result of the X-ray diffraction of the hydrate was as follows.

| Angle of diffraction (2θ°) | Relative intensity (%) |
|---|---|
| 7.22 | 52 |
| 13.40 | 29 |
| 13.80 | 27 |
| 14.20 | 30 |
| 17.75 | 100 |
| 18.95 | 63 |
| 20.40 | 44 |
| 21.30 | 69 |
| 23.97 | 45 |
| 25.70 | 53 |
| 26.32 | 46 |
| 28.00 | 23 |
| 28.65 | 36 |

Example 5

Synthesis of (6R,7R)-7-[(Z)-2-(2-aminothiazol-4-yl)-2-methoxyiminoacetoamido]-3-(1-azabicyclo[3,3,0]octane-1-io)methyl-3-cephem-4-carboxylate [compound (100)]

(i) To a suspension fo 20 g of (6R,7R)-7-amino-3-iodomethyl-3-cephem-4-carboxylic acid in 250 mℓ of acetonitrile was added dropwise 40 mℓ of bistrimethylsiylyl acetoamide under cooling with ice-water. The mixture was stirred for 1 hour.

On the other hand, to a solution of 26.3 g of (Z)-2-methoxyimino-2-(2-tritylaminothiazol-4-yl)

acetic acid in dichloromethane was added 13.2 g of phosphorous pentachloride. The mixture was stirred at room temperature. The reaction mixture was washed with water, dried over anhydrous magnesium sulfate and filtrated to give a solution of (Z)-2-methoxyimino-2-(2-tritylaminothiazol-4-yℓ) acetyl chloride in dichloromethane.

To the abovementioned solution was added slowly the solution of (Z)-2-methoxyimino-2-(2-tritylaminothiazol-4-yℓ) acetyl chloride under cooling with ice-water. The mixture was stirred at room temperature for 30 minutes to give a solution of (6R,7R)-7-[(Z)-2-(2-tritylamino thiazol-4-yℓ)-2-methoxyiminoacetoamide]-3-(1-azabicyclo [3,3,0]octane-1-io)methyl-3-cephem-4-carboxylic acid trimethylsilyl ester in a mixed solvent of acetonitrile and dichloromethane.

(ii) The solution was cooled to -20°C,and to the solution was added dropwise a solution of 5.1 g of 1-azabicyclo[3,3,0]octane in 50 mℓ of acetonitrile. The mixture was stirred for 2 hours, and then heated to room temperature.

The solvent was distilled off under reduced pressure, and to the residue was added 50 mℓ of trifluoroacetic acid containing 10% water. The mixture was vigorously stirred at room temperature for 2 hours. To the reaction mixture was added 500 mℓ of diethylether to give precipitates. The precipitates were filrated and dried over to give 41 g of a solid. The solid was crushed, and extracted with water. The extracts were concentrated, and to the residue was added ethanol. The solution was allowed to stand under cooling to give precipitates. The precipitates were

collected by filtration to give 21 g of solvate of (6R,7R)-7-[(Z)-2-(2-aminothiazol-4-yℓ)-2-methoxyimino-acetoamido]-3-(1-azabicyclo[3,3,0]octane-1-io)methyl]-3-cephem-4-carboxylate·2-trifluoroacetio acid salt [trifluoroacetate of compound(100)].

(iii) The solid was dried, and suspended in 50 mℓ of methanol. The suspension was neutralized with drops of triethylamine with stirring under cooling to give a crystal. The crystals were colledted by filtration, and dried to yield 5.2 g of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate [compound (100)] as crystals.

The compound was identified by highperformance liquid chromatography (HPLC) (column:RP-18, eluate: water-methanol).

Estimated purity was 98.2%.

Example 6

Synthesis of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate [compound (100)] according to Reaction Scheme B

(i) To a suspension of (6R,7R)-7-amino-3-iodomethyl-3-cephem-4-carboxylic acid in 2 ℓ of acetonitrile was added dropwise 435 mℓ of bistrimethylsilyl acetoamide under cooling with ice-cold water. The mixture was stirred under cooling with ice-cold water for 1 hour, and then cooled to -20°C to prepare (6R,7R)-7-bistrimethylsilylamino-3-iodomethyl-3-cephem-4-carboxylic acid trimethylsilyl ester. To the mixture was added slowly a solution of 51 g of 1-azabicyclo

[3,3,0]octane in 400 mℓ of acetonitrile with stirring to prepare a solution of (6R,7R)-7-bis trimethylsilylamino-3-(1-azabicyclo[3,3,0]octane-1-io)methyl-3-cephem-4-carboxylate in acetonitrile.

(ii) The solution was heated to room temperature, and to the solution was added at once a solution of (Z)-2-methoxyimino-2-(2-tritylaminothiazol-4-yℓ) acetyl chloride prepared from a solution of 263 g of (Z)-2-methoxyimino-2-(2-tritylaminothiazol-4-yℓ) acetic acid in 1ℓ of dichloromethane and 132 g of phosphorous pentachloride. The mixture was stirred for 1 hour and then the solvet was distilled away under reduced pressure. To the residue was added 600 mℓ of trifluoroacetic acid containing 10% water, and the mixture was stirred at room temperature for 2 hours.

Trifluoroacetic acid was distilled away, and to the residue was added 5 ℓ of diethyl ether with vigorous stirring. The precipitates were collected by filtration. The precipitates were extracted with 1.5 ℓ of water. The extracts were concentrated, and to the residue was added 2 ℓ of ethanol. The solution was allowed to stand under cooling. The precipitates were collected by filtration to give 270 g of solvate of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate·2-trifluoroacetic acid salt [trifluoroacetate of compound (100)].

(iii) The solvate was dried under vacuum to give 140 g of powder. The powder was suspended in 500 mℓ of methanol, and the suspension was neutralized with drops of triethylamine with stirring under cooling.

The solution was stirred to give precipitates as crystals. The crystals were collected by filtration, washed with methanol and dried to yield 74 g of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate [compound (100)] as crystals.

The purity of this compound was checked by high-performance liquid chromatography (HPLC) (column:RP-18, eluate: water-methanol, UV at 280mm). Estimated purity was more than 99%. The spectra date of this compound were identical with those of the compound obtained in Example 1.

Example 7

Synthesis of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate [compound (100)] according to Reaction Scheme C

(i) A solution of (6R,7R)-7-bistrimethylsilylamino-3-(1-azabicyclo[3,3,0]octane-1-io)methyl-3-cephem-4-carboxylate was prepared from a suspension of 50 g of (6R,7R)-7-amino-3-iodomethyl-3-cephem-4-carboxylic acid in 500 mℓ of acetonitrile, 110 g of bistrimethylsilylacetoamide and 13 g of 1-azabicyclo[3,3,0]octane in the same way as in (i) of Example 6.

To the solution was added under cooling a solution of an acid chloride prepared from a solution of 62 g of (2-tritylaminothiazol-4-yℓ)glyoxylic acid and 32 g of phosphorous pentachloride, and the mixture was stirred at room temperature for 30 minutes. The solvent was distilled off to give solid residue.

The residue was dissolved in 700 mℓ of methanol, and to the solution was added 7.1 g of O-methyl hydroxyamine. The mixture was stirred at room temperature for 16 hours. The solvent was distilled off under reduced pressure, and to the residue was added 150 mℓ of trifluoro acetic acid containing 10% water. The mixture was stirred vigorously at room temperature for 4 hours. The reaction mixture was added to 1ℓ of diethyl ether to give precipitates. The precipitates were collected by filtration. The precipitates were treated in the same way as in (ii) of Example 6 to give 34 g of trifluoro acetic acid salt of the captioned compound [trifluoroacetate of compound (100)]. A solution of the compound in a small amount of methanol was passed through a column of HP-20 ion-exchange resine, and eluted with a mixed solvent of acetone and water while gradually increasing the volume percentage of acetone from 0% to 40% to give 15.4 g of the powdery captioned compound [compound (100)]. The compound was identified by HPLC (column:RP-18, eluate:water-methanol, UV at 280 nm). Estimated purity was 68%.

Example 8

Synthesis of (6R,7R)-7-[(Z)-2-(2-aminothiazol-4-yℓ)-2-methoxyiminoacetoamido]-3-(1-azabicyclo[3,3,0]octane-1-io)methyl-3-cephem-4-carboxylate [compound (100)] according to Reaction Scheme D

(i) A solution of (6R,7R)-7-bistrimethylsilylamino-3-(1-azabicyclo[3,3,0]octane-1-io)methyl-3-cephem-4 carboxylate in acetonitrile was prepared from 3.4 g of (6R,7R)-7-amino-3-iodomethyl-3-cephem-4-carboxylic acid, 50 mℓ of acetonitrile and 1.0 g of 1-azabicyclo

[3,3,0]octane in the same way as in (i) of Example 6.

To the solution was added slowly at -20°C a solution of 4-chloro-3-oxo-(Z)-2-methoxyiminoacetyl chloride in dichloromethane obtained by reacting 1.6 g of 4-chloro-3-oxo-(Z)-2-methoxyimino acetic acid with 2.2 g of phosphorous pentachloride in 50 mℓ of dichloromethane at room temperature. The mixture was heated gradually to room temperature, and stirred for 1 hour. The solvent was distilled off under reduced pressure to give 11.8 g of solid residue.

(ii) The residue was dissolved in 60 mℓ of dimethylformamide, and to the solution was added 1.9 g of thiourea. The mixture was stirred at room temperature for 1 hour. The reaction mixture was added to 500 mℓ of diethylether, and then an oily substance was separated out. The etheral layer was removed by decantation, and then 50 mℓ of acetone was added. The mixture was stirred to yield a solid. The solid was dissolved in water, passed through a column of HP-20 ion-exchange resin, and eluted with a mixed solvent of water and acetone while gradually increasing the volume percentage of acetone from 0% to 40% to give 415 mg of the captioned compound (100) as white solid. The compound was identified by HPLC, and estimated purity was 75%.

Example 9

<u>Synthesis of (6R,7R)-[(Z)-2-(2-aminothiazol-4-yℓ)-2-methoxyiminoacetoamido]-3-(1-azabicylo [3,3,0]octane-1-io)methyl-3-cephem-4-carboxylate [compound (100)] according to Reaction Shecme E</u>

(i) To a suspension of 3.4 g of 7-amino-3-iodomethyl -3-cephem-4-carboxylic acid in 50 mℓ of acetonitrile was added 7.5 of bistrimethylsilyl acetoamide at room temperature, and the mixture was stirred for 1 hour. The mixture was cooled to -20°C, and to the mixture was added slowly 1.0 g of 1-azabicyclo [3,3,0]octane. Then the mixture was stirred at -20°C for 1 hour to prepare a solution of (6R,7R)-7-bis-trimethylsilylamino-3-(1-azabicyclo[3,3,0]octane-1-io)methyl-3-cephem-4-carboxylate.

To the solution was added a solution of (Z)-2-hydroxyimino-2-(2-tritylaminothiazol-4-yℓ)acetyl chloride prepared by reacting 4.2 g of (Z)-2-hydro-xymino-2-(2-tritylaminothiazol-4-yℓ)acetic acid with 2.1 g of phosphorous pentachloride in 50 mℓ of dichloromethane. The mixture was stirred at room temperature for 1 hour, and the solvent was distilled off under reduced pressure to give 13.5 g of foamy solid.

(ii) The solid was dissolved in 200 mℓ of dimethyl-sulfoxide, and to the solution were added 2.0 mℓ of methyliodide and 5.5 g of potassium carbonate. The mixture was stirred at room temperature for 2 days. The reaction mixture was added to 750 mℓ of water. The precipitates was collected by filtration, and added to 50 mℓ of trifluoroacetic acid containing 10% water. The mixture was stirred vigorously at room temperature for 3 hours. The reaction mixture was added to 500 mℓ of diethylether to give precipitates. The precipitates were collected, washed with water, and then dried. The precipitates were extracted with water, and extracts were passed a column of HP-20 ion-exchange resin, and eluted with a mixed solvent of water and acetone while

gradually increasing the volume percentage of acetone from 0% to 40% to give 320 mg of the captioned compound (100) as solid. The compound was identified by HPLC, and estimated purity was 57%.

Example 10

Synthesis of (6R,7R)-7-[(Z)-2-(2-aminothiazol-4-yl)-2-(2-carboxypropyl-2-yloxyimino)acetoamido]-3-(1-azabicyclo[3,3,0]octane-1-io)methyl-3-cephem-4-carboxylate[compound(116)]

A solution of 540 mg of diphenylmethyl(6R,7R)-7-[(Z)-2-(2-tritylaminothiazol-4-yl)-2-(2-t-butoxycarbonylpropyl-2-yloxyimino)acetoamido]-3-iodomethyl-3-cephem-4-carboxylate in 10.8 ml of dichloromethane was prepared and 85 mg of 1-azabicyclo[3,3,0]octane was added thereto at room temperature. The mixture was then stirred at room temperature for 30 minutes. After the reaction was over, 80 ml of ether was added and the resulting precipitate (328 mg) was collected by filtration. 4.2 ml of trifluoroacetic acid was added to thus collected precipitate and the mixture was stirred at room temperature for 1 hour. After the solvent was distilled away under reduced pressure, the residue was dissolved in 50 ml of phosphate buffer (pH 7.0) and passed through a column of HP-20 ion-exchange resin, amd eluted with a mixed solvent of water and acetone while gradually increasing the volume percentage of acetone from 0% to 40%. The fractions obtained with 20% to 40% acetone were collected and lyophilized to give about 38 mg of compound (116).

IR($cm^{-1}$):
1760, 1660, 1605
NMR(ppm, $D_6$ DMSO)δ:
1.48 (6H, s), 1.8-2.2 (8H, broad)
3.0-4.0 (6H, broad),
4.0-4.5 (3H, broad),
5.4 (1H), 6.75 (1H), 7.3 (1H, s),
7.28 (2H), 9.3 (1H)

Examle 11

Synthesis of (6R,7R)7-[(Z)-2-(2-aminothiazol-4-yℓ)-2-methoxyiminoacetoamido]-3-(1-azabicyclo[4,3,0]nonane-1-io)methyl-3-cephem-4-carboxylate [compound(102)]

A suspension of 468 mg of diphenylmethyl (6R, 7R)-7-[(Z)-2-(2-tritylaminothiazol-4-yℓ)-2-methoxyiminoacetoamido]-3-iodomethyl-3-cephaem-4-carboxylate in 30 mℓ of ether was prepared. After the addition of 125 mg of 1-azabicyclo[4,3,0]nonane therto at room temperature, the mixture was stirred at room temperature for 1.5 hours. The resulting precipitate was filtered and 3 mℓ of trifluoroacetic acid was added to the obtained cake (274 mg) and the mixture was stirred at room temperature for 1.5 hours. Trifluoroacetic acid was removed by distillation at or below room temperature (20°C) under vacuum conditions. When 20 mℓ of ether was added to the residue and mixed vigorously, an oily substance was separated out. After the removal of the ethereal layer by decantation, the oily substance was dissolved in a small amount of methanol. The purification was carried out by passing the solution through a column of HP-20 ion-exchange resin

and eluting a mixed solvent of water and methanol while gradually increasing the volume percantege of methanol from 0% to 40%. The fractions eluted with 20% ~ 30% ethanol were collected and lyophilized to obtain 40 mg of powder containing the compound (102).

MMR (ppm $D_6$ DMSO) δ:

| | |
|---|---|
| 1.8-2.2 | (10H broad) |
| 3.1-3.6 | (4H broad) |
| 3.6 | (2H multiplet) |
| 3.95 | (2H multiplet) |
| 4.0 | (3H singlet) |
| 5.35 | (1H doublet) |
| 5.85 | (1H doublet) |
| 7.05 | (1H singlet) |

Example 12

Snythesis of (6R,7R)-7-[(Z)-2-(2-aminothiazol-4-yℓ)-2-methoxyiminoacetoamido]-3-(1-azabicyclo[4,4,0]decane-1-io)methyl-3-cephem-4-carboxylate [compound(104)]

The reaction was carried out according to Example 10 between 468 mg of diphenylmethyl (6R,7R)-7-[(Z)-2-(2-tritylaminotiazole-4-yℓ)-2-methoxyimino aceto-amido]-3-iodomethyl-3-cephem-4-carboxylate and 140 mg of 1-azabicyclo[4,4,0]decane in ether and the reaction product was treated likewise with trifluoroacetic acid and ether to give 45 mg of crude product. After the product was dissolved in ethanol, the purification was carried by passing the solution through a column of HP-20 ion-exchange resin and eluting a mixed solvent of water and methanol. The fractions eluted with the mixed solvent of water and

methanol (water: methanol=70:30) gave about 50 mg of powder containing the compound (104).

IR ($cm^{-1}$):
1760, 1540, 1130, 1090.

Example 13

Synthesis of (6R,7R)-7-[(Z)-2-(2-aminothiazol-4-yℓ)-2-(2-carboxypropyl-2-yloxyimino) acetoamido]-3-(1-azabicycl0[4,3,0]nonane-1-io) methyl-3-cephem-4-carboxylate[compound(134)]

A solution of 270 mg of diphenylmethyl (6R,7R)-7-[(Z)-2-(2-tritylaminothiazol-4-yℓ)-2-(2-t-butoxycarbonylpropyl-2-yℓ oxyimino)acetoamido]-3-iodomethyl-3-cephem-4-carboxylate in 25 mℓ of ether was prepared, and 50 mg of 1-azabicyclo[4,3,0]nonane was added thereto and the mixture was stirred at 16°C for 45 minutes. The reaction product was diluted with 50 mℓ of ether and filtered. 4.5 mℓ of 90% trifluoroacetic acid was added to the obtained precipitate (about 200 mg) and the mixture was stirred at room temperature (20°C) for 1 hour. Trifluoroacetic acid was distilled away under reduced pressure and the residue was dissolved in phosphate buffer (pH 7.6). The solution was passed through the column of HP-20 ion-exchange resin, and eluted with a mixed solvent of acetone and water while increasing the volume ratio of acetone from 0% to 40%. The fractions eluted at aceton's volume ratio of 10% to 20% were collected and freeze-dried to obtain 24 mg of powder containing the compound (134).

IR ($cm^{-1}$): 1760, 1640, 1540, 1130, 1090.

Example 14

Synthesis of (6R,7R)-7-[(Z)-2-(2-aminothiazol-4-yl)-2-methoxyiminoacetoamido]-3-(1-aza-8-methyl[3,3,0]octane-1-io)methyl-3-cephem-4-carboxylate[compound(106)]

A suspension of 702 mg of diphenylmethyl (6R,7R)-7-[(Z)-2-(2-tritylaminothiazol-4-yl)-2-methoxyimino-acetoamido]-3-iodomethyl-3-cephem-4-carboxylate in 45 mℓ of ether was prepared and 187.5 mg of 1-aza-8-methyl[3,3,0]octane was added thereto. The mixture was stirred at room temperature for 3 hours. The resulting precipitate was filtered and after the addition of 4.5 mℓ of trifluoroacetic acid and 0.45 mℓ of water to 692 mg of the obtained cake, the mixture was stirred at room temperature for 1 hour. Trifluoroacetic acid was distilled away at room temperature under reduced pressure and the residue was pulverized in ether, collected by filtration, and dissolved in a small amount of methanol. The solution was passed through a column of HP-20 ion-exchange resin, and eluted with the mixed solvent of water and methanol while increasing the amount of methanol by the volume ratio from 0% to 40% in regular succession. The fractions eluted at methanol's volume ratio of 20% to 40% were collected and lyophilized to obtain 42 mg of powder containing the compound (106).

NMR (ppm $D_2O$) $\delta$:

| | |
|---|---|
| 1.5 | (3H singlet) |
| 2.1-2.3 | (8H broad) |
| 3.3-3.9 | (6H broad) |
| 3.9-4.2 | (2H multiplet) |

| | |
|---|---|
| 4.0 | (3H singlet) |
| 5.35 | (1H doublet) |
| 5.85 | (1H doublet) |
| 6.75 | (1H singlet) |
| 7.1 | (1H singlet) |

Example 15

Synthesis of (6R,7R)-7-[(Z)-2-(2-aminothiazol-4-yl)-2-methoxyiminoacetoamido]-3-(1-aza-8-cyanobicyclo[3,3,0]octane-1-io)methyl-3-cephem-4-carboxylate[compound(108)]

A suspension was prepared by suspending 420 mg of diphenylmethyl (6R,7R)-7-[(Z)-2-(2-tritylamino-thiazol-4-yl)2-methoximino acetoamido-3-iodomethyl-3-cephem-4-carboxylate in 42 ml of anhydrous ether. 114 mg of 1-aza-8-cyanobicyclo[3,3,0]octane was added to this suspension and the mixture was stirred at room temperature for 1.5 hours. 42 ml of ether was added to the reaction mixture and the resulting precipitate was filtered. 3.5 ml of trifluoroacetic acid was added to 160 mg of the cake obtained and the mixture was stirred at room temperature for 1 hour. As done in Example 10, after the removal of trifluoroacetic acid under vacuum, the residue was purified chromatographically with a column of HP-20 ion-exchange resin over a mixed solvent of acetone and water. Thus 7.5 mg the compound (108) was obtained from the fractions elueted at the ratio of acetone:water 2:8.

IR ($cm^{-1}$):

2230, 1770, 1535, 1140, 1085.

Example 16

Synthesis of (6R,7R)-7-[(Z)-2-(aminothiazol-4-yℓ)-2-(2-carboxypropyl-2-yloxyimino)acetoamido]-3-(1-aza-8-hydroxymethyl[3,3,0]octane-1-io)methyl-3-cephem-4-carboxylate[compound(138)]

A solution was prepared by dissolving 270 mg of diphenylmethyl (6R,7R)-7-[(Z)-2-(2-tritylaminothiazol-4-yℓ)-2-(2-t-butoxycarbonylpropyl-2-yloxyimino)acetoamido]-3-iodomethyl-3-cephem-4-carboxylate in 25 mℓ of ether and 150 mg of 1-aza-8-hydroxymethyl[3,3,0]octane was added thereto. The mixture was stirred at room temperature for 2 hours. After the reaction is completed, the precipitate was collected by filtration and after the addition of 2.5 mℓ of trifluoroacetic acid thereto, the mixture was stirred at room temperature for 2 hours. Trifluoroacetic acid was removed by distillation under reduced pressure and the residue was crushed to particles, which were then subjected to filtration. A solution of resulting cake of methanol was chromatographically purified by being made to pass through a column of HP-20 ion-exchange resin over the mixed solvent of water-acetone with the volume ratio of acetone increasing from 0% to 50% gradually. The eluted fractions were collected and lyophilized to give 14 mg of the compound (138).

IR ($cm^{-1}$):
3400, 1760, 1600, 1535, 1140.

Example 17

Synthesis of (6R,7R)-7-[(Z)-2-(2-aminothiazol-4-yl)-2-methoxyiminoacetoamido]-3-(1-azabicyclo[2,2,1]heptane-1-io)methyl-3-cephem-4-carboxylate [compound(200)]

A suspension was prepared by suspending 468 mg of diphenylmethyl (6R,7R)-7-[(Z)-2-(2-tritylaminothiazol-4-yl)-2-methoxyiminoacetoamido]-3-iodomethyl-3-cephem-4-carboxylate in 30 ml of ether. After the addition of 97 mg of 1-azabicyclo[2,2,1]heptane thereto, the mixture was stirred at room temperature for 1 hour. The precipitate was filtered and 3 ml of trifluoroacetic acid and 0.3 ml of water were added to the obtained cake. The mixture was stirred at room temperature for 1 hour. Trifluoroacetic acid was then distilled away at or below room temperature under vacuum conditions. Ether was added to the residue, which was then crushed. The resulting particles were collected by filtration and dissolved in a small amount of methanol. The solution was passed through a column of HP-20 ion-exchange resin and eluted with a mixed solvent of water and methanol while gradually increasing the volume ratio of methanol from 0% to 40%. The fractions eluted at the methanol:water volume ratio changing from 2:8 to 3:7 were collected and lyophilized to obtain 81 mg of the compound (200).

NMR(ppm, $D_2O$)$\delta$:

| | |
|---|---|
| 2.1 | (4H broad) |
| 3.0 | (1H broad) |
| 3.3 | (2H multiplet) |
| 3.3-3.6 | (4H broad) |

| | |
|---|---|
| 3.7-3.6 | (2H broad) |
| 4.0 | (3H singlet) |
| 5.3 | (1H doublet) |
| 5.85 | (1H doublet) |
| 6.87 | (1H singlet) |

Example 18

Synthesis of (6R,7R)-7-[(Z)-2-(2-aminothiazol-4-yℓ)-2-(2-carboxypropyl-2-yloxyimino)acetoamido]-3-(1-azabicyclo[2,2,1]heptane-1-io)methyl-3-cephem-4-carboxylate[compound(202)]

A solution of 540 mg of diphenylmethyl (6R,7R)-7-[(Z)-2-(2-tritylaminothiazol-4-yℓ)-2-(2-t-butoxycarbonylpropyl-2-yloxyimino)acetoamido]-3-cephem-4-carboxylate in 50 mℓ of ether was prepared and 97 mg of 1-azabicyclo[2,2,1]heptane was added thereto. The mixture was stirred at room temperature for 1 hour and, after the reaction was over, the resulting precipitate (440 mg) was obtained by filtration. Then, 3.5 mℓ of trifluoroacetic acid and 0.35 mℓ of water were added thereto and the admixture was stirred vigorously at room temperature for 1.5 hours. Both trifluoroacetic acid and water were distilled away under reduced pressure and the residue was pulveried in ethanol, subjected to filtration, and dissolved in a mixed solvent of water and methanol. The solution was purified through a column of HP-20 ion-exchange resin over a mixed solvent of water and methanol while gradually increasing the volume ratio of methanol. The fractions eluted at the volume ratios of methanol from 10% to 20% were collected and freeze-dried to obtain 68 mg of the compound (202).

IR ($cm^{-1}$)
1760, 1660, 1160.

Example 19

Synthesis of (6R,7R)-7-[(Z)-2-(2-aminothiazol-4-yℓ)-2-methoxyiminoacetoamido]-3-(1-aza-3-oxo-bicyclo[2,2,2]octane-1-io)methyl-3-cephem-4-carboxylate[compound(300)]

To a suspension of 468 mg of diphenylmethyl (6R,7R)-7-[(Z)-2-(2-tritylaminothiazol-4-yℓ)-2-methoxyiminoacetoamido]-3-iodomethyl-3-cephem-4-carboxylate was added 127 mg of 1-aza-3-oxobicyclo [2,2,2]octane. The mixture was stirred at room temperature for 1 hour. The reaction mixture was subjedted to filtration. To the precipitates was added 3 mℓ of trifluoroacetic acid. The mixture was stirred at room temperature for 2 hours. Trifluoroacetic acid was distilled off under reduced pressure, and the residue was washed with ether, and extracted with water. The extracts was passed through a column of HP-20 ion-exchange resin, and eluted with a mixed solvent of water and methanol while gradually increasing the volume percentage of methanol from 0% to 50%. The active fractions were collected and lyophilized to yield the compound (300).

IR ($cm^{-1}$): 1768
NMR (ppm, $D_2O$) δ:

| | |
|---|---|
| 1.7-2.3 | (6H, broad) |
| 2.7-3.0 | (1H, broad) |
| 3.2-4.0 | (6H, broad) |
| 4.00 | (3H, s) |
| 5.36 | (1H, d) |

| | |
|---|---|
| 5.86 | (1H, d) |
| 7.01 | (H, s) |

Example 20

Synthesis of (6R,7R)-7-[(Z)-2-(2-aminothiazol-4-yl)-2-methoxyiminoacetoamido]-3-(1-aza-3-hydroxybicyclo[2,2,2]octane-io)methyl-3-cephem-4-carboxylate[compound(302)]

From 1-aza-3-hydroxybicyclo[2,2,2]octane, compound (302) was obtained by the same operation as in Example 19.

IR ($cm^{-1}$): 1765

NMR (ppm, $D_2O$) $\delta$:

| | |
|---|---|
| 1.7-2.3 | (6H, broad) |
| 2.9-4.0 | (6H, broad) |
| 4.00 | (3H, s) |
| 5.35 | (1H, d) |
| 5.86 | (1H, d) |
| 7.02 | (1H, s) |

Example 21

Synthesis of (6R,7R)-7-[(Z)-2-(2-aminothiazol-4-yl)-2-methoxyiminoacetoamido]-3-(1-aza-4-cyanobicyclo[2,2,2]octane-1-io)methyl-3-cephem-4-carboxylate[compound(318)]

From 1-aza-4-cyanobicyclo[2,2,2]octane, compound (318) was obtained by the same operation as in Example 19.

IR ($cm^{-1}$): 1770

NMR (ppm, $D_2O$) $\delta$:

| | |
|---|---|
| 2.1-2.6 | (6H, broad) |
| 3.2-3.7 | (6H, broad) |
| 4.00 | (3H, s) |
| 5.36 | (1H, d) |
| 5.86 | (1H, d) |
| 7.01 | (1H, s) |

Example 22

Synthesis of (6R,7R)-7-[(Z)-2-(2-aminothiazol-4-yℓ)-2-methoxyiminoacetoamido]-3-(1-aza-3-chlorobicyclo[2,2,2]octane-1-io)methyl-3-cephem-4-carboxylate[compound(304)]

From 1-aza-3-chlorobicyclo[2,2,2]octane, the compound (304) was obtained by the same operation as in Example 19.

IR ($cm^{-1}$): 1770, 1660, 1610, 1030.

Example 23

Synthesis of (6R,7R)-7-[(Z)-2-(2-aminothiazol-4-yℓ)-2-methoxyiminoacetoamido]-3-(1-aza-4-ethoxycarbonylbicyclo[2,2,2]octane-1-io)methyl-3-cephem-4-carboxylate[compound(320)]

From 1-aza-4-ethoxycarbonylbicyclo[2,2,2]octane, the compound (320) was obtained by the same operation as in Example 19.

IR ($cm^{-1}$): 1765, 1730, 1670, 1620, 1025.

Example 24

Synthesis of (6R,7R)-7-[(Z)-2-(2-aminothiazol-4-yℓ)-2-methoxyiminoacetoamido)-3-(1-aza-4-hydroxymethylbicyclo[2,2,2]octane-1-io)methyl--3-cephem-4-carboxylate[compound(322)]

From 1-aza-4-hydroxymethylbicyclo[2,2,2]octane, the compound (322) was obtained by the same operation as in Example 19.

IR ($cm^{-1}$): 1763, 1665, 1620, 1020.

Example 25

Synthesis of (6R,7R)-7-[(Z)-2-(2-aminothiazol-4-yℓ)-2-methoxyiminoacetoamido]-3-[1-aza-4-(2,5-dihydro-2-methyl-5-oxotriazin-3-yℓ) thiomethylbicyclo[2,2,2]octane-1-io]methyl-3-cephem-4-carboxylate[compound(324)]

From 1-aza-4-(2,5-dihydro-2-methyl-5-oxotriazin-3-yℓ)thiomethylbicyclo[2,2,2]octane, the compound (324) was obtained by the same operation in Example 19.

IR ($cm^{-1}$): 1780, 1665, 1630, 1620, 1040.

Example 26

In vitro antibacterial activity

In vitro antibacterial activity was determined by the two-fold agar-plate dillution method.

One loopful of an overnight culture of each test starin in Trypticase-soy broth ($10^6$ viable cells per mℓ) was streaked on heart infusion agar (Muller-Hinton Agar) containing graded concentrations of representative test compound, and the minimal inhibitory concentration (MIC) was expressed in terms of μg/mℓ after incubation at 37°C for 16 hours. The results are shown in Table-I.

Table-I, MIC (μg/mℓ)

| | Compound(100) | Comparative compound A | Comparative compound B |
|---|---|---|---|
| Staphyrococcus aureus FDA209JC-1 | 0.39 | 1.56 | 3.12 |
| Staphyrococcus Terajima | 0.39 | 1.56 | 0.78 |
| Staphyrococcus aureus MS 353 | 0.20 | 1.56 | 0.20 |
| Staphyrococcus pyogenes COOK | <0.0063 | 0.0063 | 0.025 |
| Escherichia coli K-12C-600 | 0.05 | 0.20 | 0.05 |
| Salmonera typhimurium IID 971 | 0.05 | 0.39 | 0.39 |
| Salmonera schottmuelleri 8001 | <0.0063 | 0.025 | 0.0063 |
| Butilus subtilis ATCC6633 | 0.39 | 0.78 | 0.78 |
| Pseudomonas aeruginosa IFO3445 | 1.56 | 1.56 | 12.5 |
| Pseudomonas aeruginosa NCTC10490 | 0.025 | 0.39 | 0.39 |
| Pseudomonas aeruginosa PA01 | 0.10 | 0.10 | 12.5 |
| Enterobacter cloacae 963 | 0.05 | 0.20 | 0.20 |
| Micrococcus luteus ATCC9341 | <0.0063 | 0.0063 | 0.39 |

Comparative compound A:

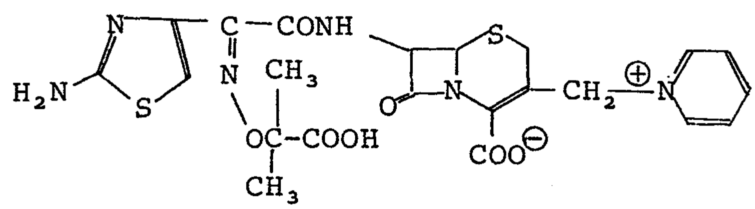

Comparative compound B:

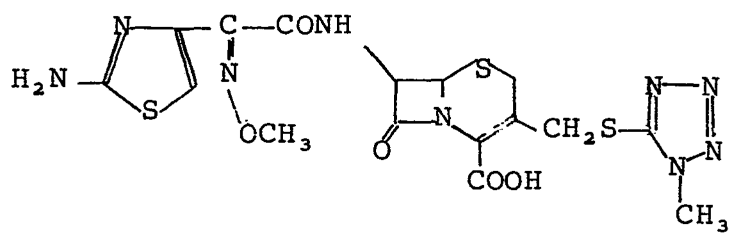

Example 27

In vitro antibacterial activities of other compounds of the present invention were determined in the same way as in Example 26.

The results are shown in Table-II.

Table-II, MIC (μg/mℓ)

| | Compound (106) | Compound (104) | Compound (200) | Compound (300) | Compound (302) | Compound (116) |
|---|---|---|---|---|---|---|
| Staphyrococcus Terajima | 0.2 | 0.39 | 0.2 | 0.2 | 0.1 | 0.78 |
| Salmonera paratyphi 1015 | 0.025 | 0.025 | 0.025 | 0.0125 | 0.0125 | 0.0125 |
| Serratia marcescens IAM1184 | 0.05 | 0.025 | 0.025 | 0.0125 | 0.0125 | 0.0125 |
| Pseudomonas aeruginosa IF03445 | 0.78 | 1.56 | 1.56 | 0.39 | 0.39 | 0.2 |
| Proleus morgani IF03848 | 0.025 | 0.0125 | 0.0063 | >0.0063 | 0.0063 | 0.0063 |
| Proleus vulgaria OX-19 | 0.025 | 0.025 | 0.05 | 0.025 | 0.025 | 0.0125 |
| Proleus vulgaris IFO 3850 | 0.0125 | 0.0125 | 0.0125 | 0.0125 | 0.0063 | 0.0063 |
| Enterobacter cloacae 963 | 0.2 | 0.1 | 0.05 | 0.025 | 0.025 | 0.05 |

Example 28

In vivo antibacterial activity

Experiment was performed in ICR strain male mice (5W) weighing 22 to 25 g. The animals were used in a group of 10 per regimen. Test organism, E.coli 81, was cultured on Mueller-Hinton agar over night at 37°C and suspended in saline to be one minimal lethal dose ($5 \times 10^5$ cells/head). One mililitre of the bacterial suspension was injected intrapenetoreally

and, after 15 minutes, mice were treated subcutaneously with various doses of compound (100) and comparative compound B. Survival mice were counted for 4 days and final observation was carried out on day 4. The results are shown in Table-III.

Table-III, MIC (μg/mℓ)

| Dose (mg/head) | Survivals percent (%) on day 4 | |
|---|---|---|
| | Compound(100) | Comparative compound B |
| 0.5 | 100 | 40 |
| 0.25 | 100 | 40 |
| 0.125 | 100 | 40 |
| 0.063 | 50 | 30 |
| 0.032 | 40 | 20 |
| 0.016 | 40 | 0 |
| control (saline) | 0 | 0 |

Example 29

Duration of antibiotic activity of compound (100)

The duration of antibiotic activity of compound (100) was determined.

Experiment was performed in ICR strain male mice (5W). The animals were used in a group of 10 per regimen. A solution of E.coli 81 was injected intrapenetreally at a dose of 5 × $10^5$ cell/head. Before one or three hours, the compound (100) was injected subcutaneously at a dose of 0.5 mg/head. Survival mice were counted on day 4 after injection. The results are shown in Table-IV.

Table IV

| Compound | Survival percent (%) | | MIC (μg/mℓ) in E.coli 81 |
|---|---|---|---|
| | Injection before one hour | Injection before three hours | |
| Compound (100) | 100 | 100 | 0.025 |
| Comparative compound | 90 | 0 | 0.0025 |

As clearly be understood, the compound (100) of the present invention is excellent in duration of antibabiotic activity.

Example 30

<u>Dry powder for injection in solution</u>

500 mg of crystalline (6R,7R)-7-[(Z)-2-(2-aminothiazol-4-yℓ-2-methoxyiminoacetoamido]-3-(1-azabicyclo-[3,3,0]octane-1-io)methyl-3-cephem-4-carboxylate was filled aseptically into glass vials under a blanket of nitrogen. The vials were closed using rubber discs. The product is intended for reconstitution by dissolving suitable sterile vehicle shortly before injection.

CAPABILITY OF EXPLOITATION IN INDUSTRY

The cephalosporin derivative of the present invention or its salt or ester thereof has a highly effective antibacterial activity against the wide varieties of Gram-positive and Gram-negative bacteria and is very useful for the treatment or prevention of bacterial infectious diseases.

WHAT WE CLAIM IS:

1. A cephalosporin derivative of the formula (1)

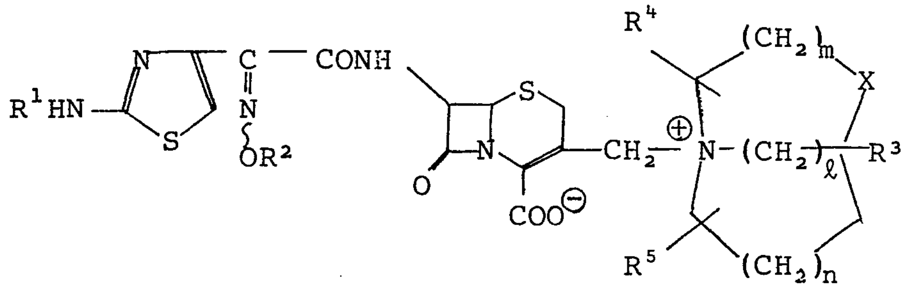

(1)

wherein $R^1$ represents a hydrogen atom or a protective group; $R^2$ represents a hydrogen atom, a substituted or unsubstituted alkyl group, a substituted or unsubstituted cycloalkyl group, or a substituted or unsubstituted hetero ring group; $R^3$ represents a hydrogen atom, a substituted or unsubstituted alkyl group, a cyano group, a carboxyl group, an alkoxycarbony group, a carbamoyl group, a carbamoyloxy group, a hetero ring group, a hydroxyl group, an alkoxyl group or a halogen atom; $R^4$ and $R^5$ are identical or different and each represents a hydrogen atom or an alkyl group; $\ell$ represents 0, 1 or 2; m and n respectively represents 1 or 2 when $\ell$ is 0; m and n respectively represent 0 when $\ell$ is 1 or 2; X represents a methylene group when $\ell$ is 0 or 1; and X represents a methylene group, a methylene group having a substituent, or an oxo group when $\ell$ is 2, provided that when $R^3$, $R^4$ and $R^5$ respectively represent a hydrogen atom, X is not a methylene group,

or its salt or ester thereof.

2. The cephalosporin derivative or its salt or ester thereof of claim 1 which is represented by the formula (1-1).

0172919

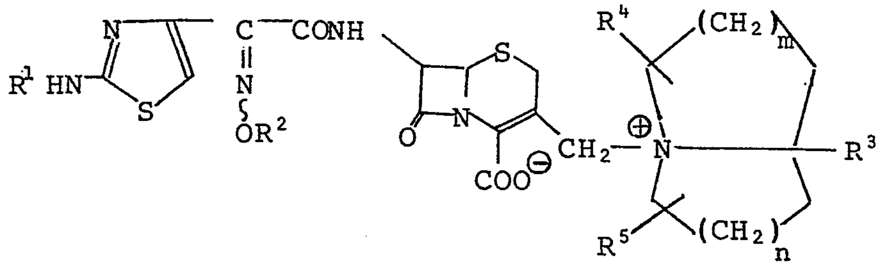 (1-1)

wherein $R^1$, $R^2$, $R^3$, $R^4$, $R^5$, m and n are as defined above.

3. The cephalosporin derivative or its salt or ester thereof of claim 1 which is represented by the formula (1-2)

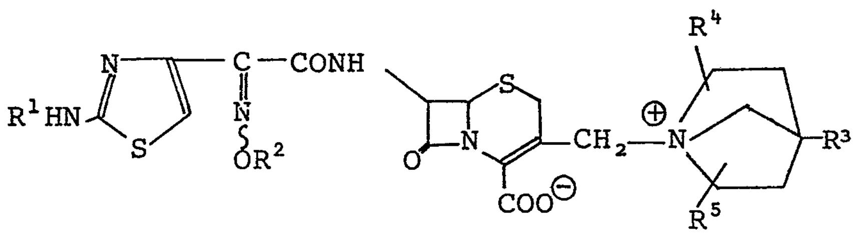 (1-2)

wherein $R^1$, $R^2$, $R^3$, $R^4$ and $R^5$ are as defined above.

4. The cephalosporin derivative or its salt or ester thereof of claim 1 which is represented by the formula (1-3)

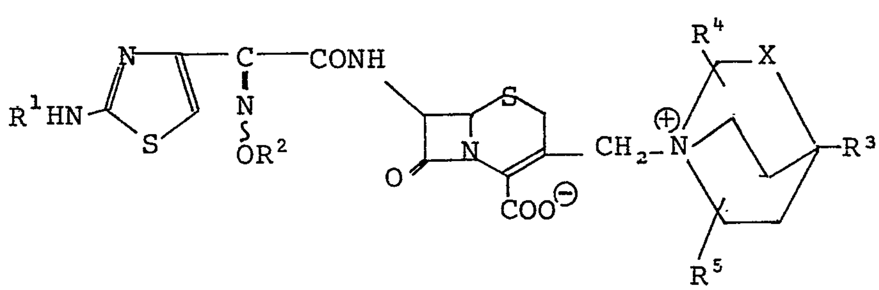 (1-3)

wherein $R^1$, $R^2$, $R^3$, $R^4$, and $R^5$ are as defined above, and X represents a methylene group, a methylene group

having a substituent, or an oxo group, provided that when $R^3$, $R^4$ and $R^5$ respectively represent a hydrogen atom, X is not a methylene group.

5. The cephalosporin derivative or its salt or ester thereof of claim 1 where in $R^2$ is a lower alkyl group or a lower alkyl group substituted by a carboxyl group.

6. The cephalosporin derivative or its salt or ester thereof of claim 1 wherein $R^3$ is a hydrogen atom, substituted or unsubstituted methyl group, a cyano group or an alkoxycarbonyl group.

7. The cephalosporin derivative or its salt or ester thereof of claim 1 wherein $R^4$ and $R^5$ are a hydrogen atom respectively.

8. The cephalosporin derivative or its salt or ester thereof of claim 1 wherein the salt is represented by the formulae (1') or (1'')

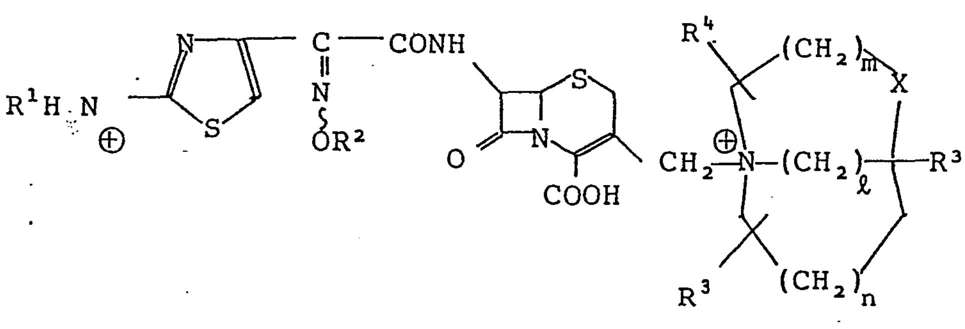

(1')

$An_1^{\ominus}$, $\frac{1}{2}An_2^{2\ominus}$ or $\frac{1}{3}An_3^{3\ominus}$

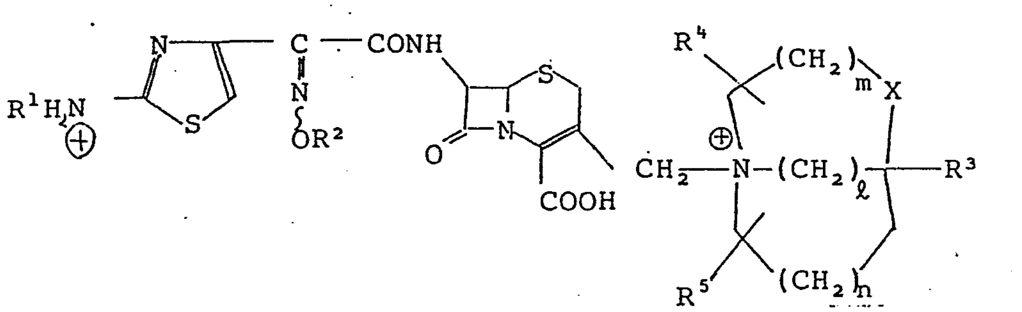

(1")

$2An_1^{\ominus}$, $An_2^{2\ominus}$ or $\frac{2}{3}An_3^{3\ominus}$

wherein $R^1$, $R^2$, $R^3$, $R^4$, $R^5$, $\ell$, m, n and X are as defined above, $An_1^{\ominus}$ is an anion of monobasic acid, $An_2^{2\ominus}$ is an anion of dibasic acid and $An_3^{3\ominus}$ is an anion of tribasic acid.

9. The cephalosporin derivative or its salt or ester thereof of claim 1 which is in the form of crystal or its hydrate.

10. A process for preparing a cephalosporin derivative of the formula (1)

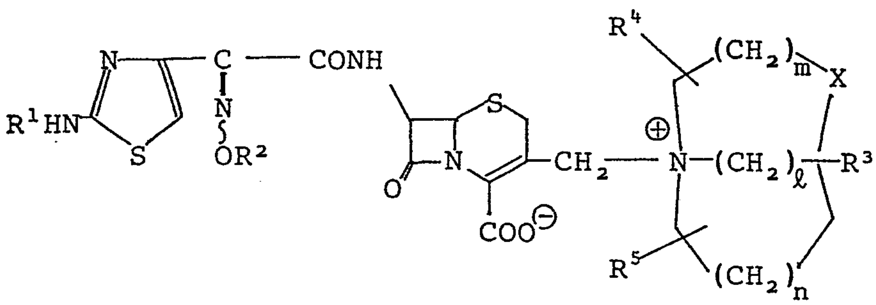

(1)

wherein $R^1$, $R^2$, $R^3$, $R^4$, $R^5$, X, $\ell$, m and n are as defined above,

or its salt or ester thereof, which comprises reacting a cepharosporin compound of the formula (2)

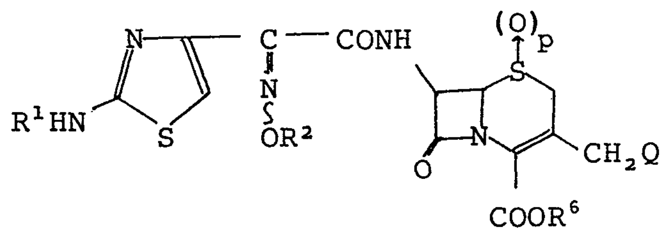

(2)

wherein $R^1$ and $R^2$ are as defined above, $R^6$ represents a hydrogen atom or a protective group, Q represents an acyloxy group, a carbamoyloxy group, or a halogen atom, and p represents 0 or 1,

or its salt with a compound of the formula (3)

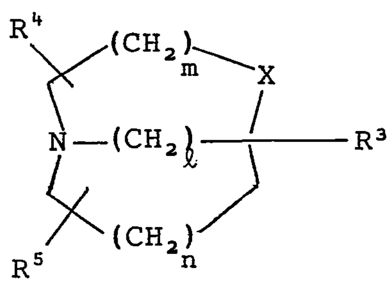

(3)

wherein $R^3$, $R^4$, $R^5$, X, $\ell$, m and n are as defined above,

or its salt, if necessary, subjecting the reaction product to deprotection, salt-forming reaction, esterification or reduction.

11. The process for preparing a cephalosporin derivative, its salt or ester thereof of claim 10 wehrein $R^6$ in the formula (2) in a trialkylsilyl group

12. A process for preparing a cephalosporin derivative of the formula (1)

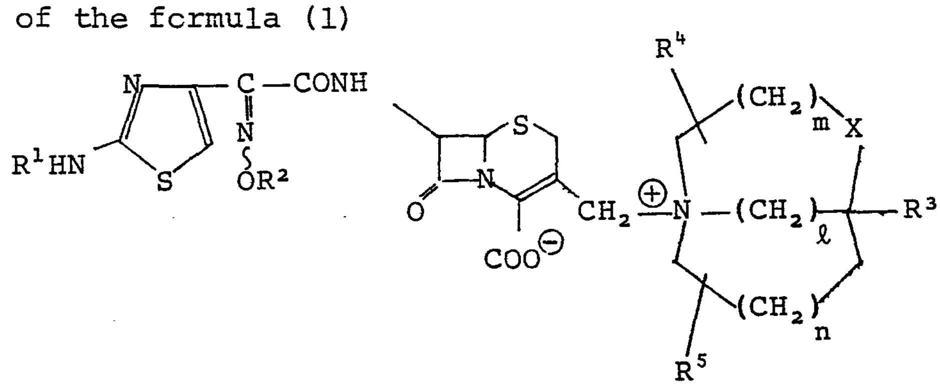

(1)

wherein $R^1$, $R^2$, $R^3$, $R^4$, $R^5$, X, $\ell$, m and n are as defined above,

or its salt or ester thereof, which comprises reacting a compound of the formula (4)

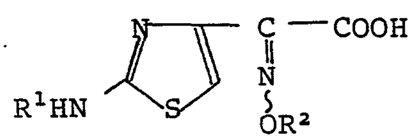

(4)

wherein $R^1$ and $R^2$ are as defined above,

its salt or reactive derivative thereof with a compound of the formula (5)

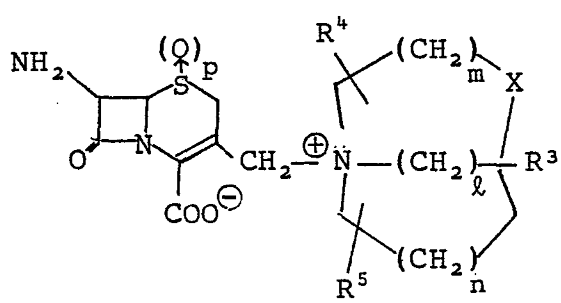

(5)

wherein $R^3$, $R^4$, $R^5$, X, $\ell$, m, n and p are as defined above,

or its salt or ester thereof or reactive derivative thereof, if necessary, subjecting the reaction product to deprotection, salt-forming reaction, esterification or reduction.

13. A process for preparing a cephalosporin derivative of the formula (1)

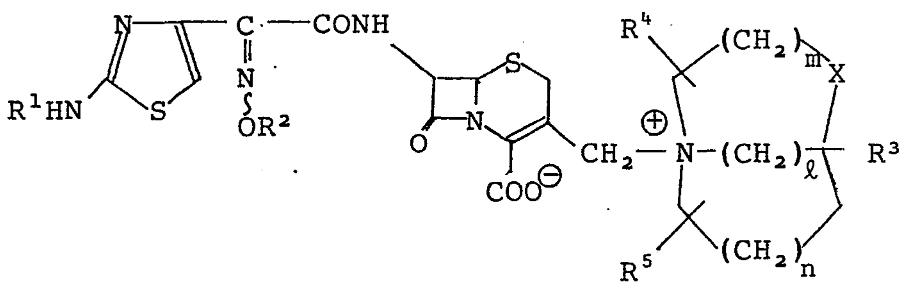

(1)

wherein $R^1$, $R^2$, $R^3$, $R^4$, $R^5$, X, ℓ, m and n are as defined above,

or its salt or ester thereof, which comprises reacting a compound of the formula (6)

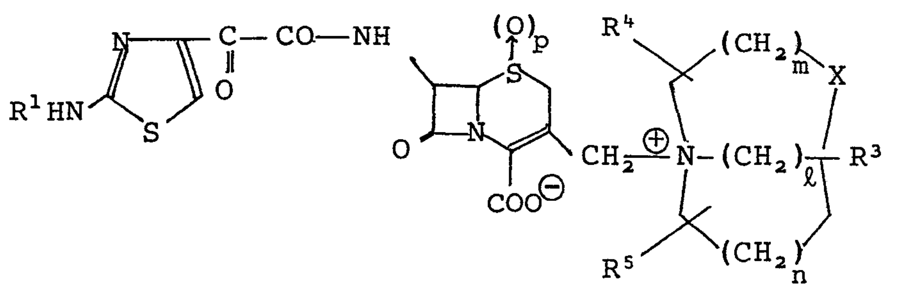

(6)

wherein $R^1$, $R^3$, $R^4$, $R^5$, X, ℓ, m, n and p are as defined above,

or its salt or ester thereof with a compound of the formula (7)

$$H_2N - OR^2 \quad (7)$$

wherein $R^2$ is as defined above,

or its protected compound or salt thereof, if necessary, subjecting the reaction product to deprotection, salt-forming reaction, esterification or reduction.

14. A process for preparing a cephalosporin derivative of the formula (1)

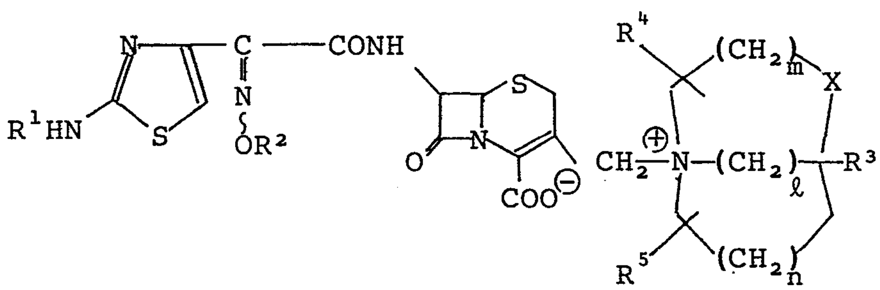

(1)

wherein $R^1$, $R^2$, $R^3$, $R^4$, $R^5$, X, ℓ, m and n are as defined above,

or its salt or ester thereof, which comprises reacting a compound of the formula (8)

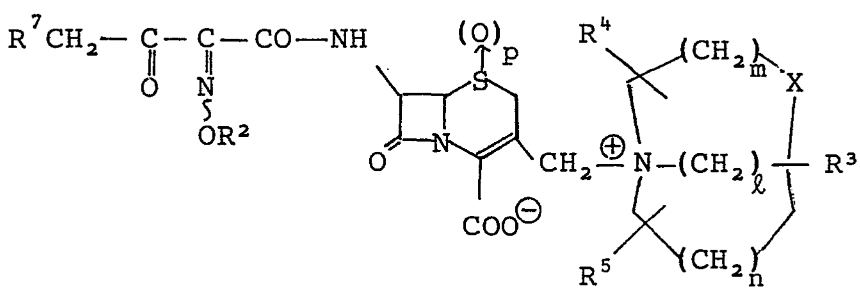

(8)

wherein $R^2$, $R^3$, $R^4$, $R^5$, $R^7$, X, ℓ, m n and p are as defined above and $R^7$ represents a halogen atom,

or its salt or ester thereof with a compound of the formula (9)

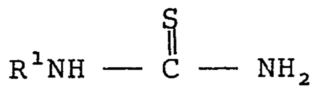

(9)

wherein $R^1$ is as defined above,

or its salt, if necessary, subjecting the reaction product to deprotection, salt-forming reaction, esterification or reduction.

15. A process for preparing a cephalosporin derivative of the formula (I'''')

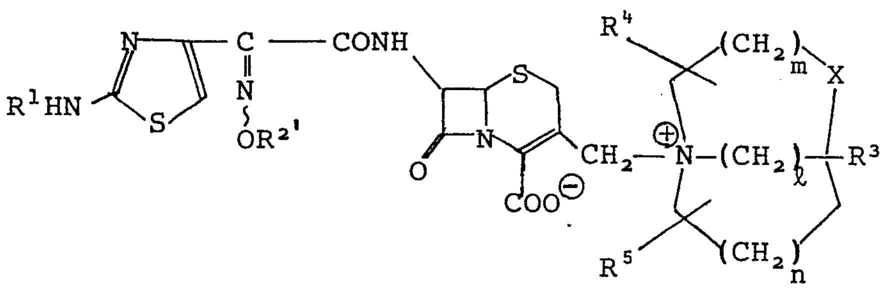

(I'''')

wherein $R^1$, $R^2$, $R^3$, $R^4$, $R^5$, X, $\ell$, m and n are as defined above,

or its salt or ester thereof, which comprises reacting a compound of the formula (9)

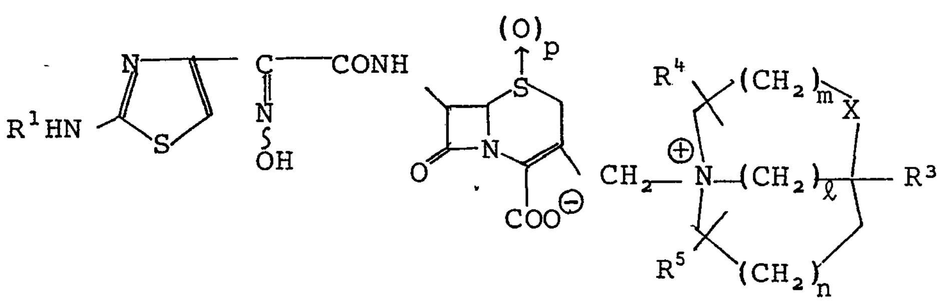

(I''')

wherein $R^1$, $R^2$, $R^3$, $R^4$, $R^5$, X, $\ell$, m, n and p are as defined above,

or its salt or ester thereof with a compound of the formula (10)

$$Hal — R^{2'} \qquad (10)$$

wherein Hal represents a halogen atom, $R^2$ is as defined above,

a dialkylsulfate or a diazoalkane, if necessary, subjecting the reaction product to deprotection, salt-forming reaction, esterification, or reduction.

16. An antibacterial composition which comprises the cepharosporin derivative or its salt or eater thereof of claim 1 as active ingredient and, if necessary, a pharmaceutically acceptable carrier.

# INTERNATIONAL SEARCH REPORT

0172919

International Application No. PCT/JP85/00101

**I. CLASSIFICATION OF SUBJECT MATTER** (if several classification symbols apply, indicate all) [3]

According to International Patent Classification (IPC) or to both National Classification and IPC

Int.Cl$^4$ C07D 519/00, 501/46, A61K 31/545

**II. FIELDS SEARCHED**

Minimum Documentation Searched [4]

| Classification System | Classification Symbols |
|---|---|
| IPC | C07D 519/00, 501/46, A61K 31/545 |

Documentation Searched other than Minimum Documentation to the Extent that such Documents are Included in the Fields Searched [5]

**III. DOCUMENTS CONSIDERED TO BE RELEVANT** [14]

| Category* | Citation of Document, [16] with indication, where appropriate, of the relevant passages [17] | Relevant to Claim No. [18] |
|---|---|---|
| P | Chemical Abstracts, Vol. 102, No.7, 18 February 1985 (18. 02. 85) (Columbus. Ohio. U. S. A.) "Cephalosporin derivatives" Abstract No. 61997b | 1 |

* Special categories of cited documents: [15]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| May 23, 1985 (23. 05. 85) | June 3, 1985 (03. 06. 85) |
| International Searching Authority [1] | Signature of Authorized Officer [20] |
| Japanese Patent Office | |